(19) Europäisches Patentamt
European Patent Office
Office européen des brevets

(11) Veröffentlichungsnummer: **0 128 482**
**B1**

(12) # EUROPÄISCHE PATENTSCHRIFT

(45) Veröffentlichungstag der Patentschrift: **31.01.90**

(51) Int. Cl.⁴: **A 61 K 47/00, A 61 K 9/20**

(21) Anmeldenummer: **84106271.4**

(22) Anmeldetag: **01.06.84**

(54) **Neue galenische Zubereitungsformen von oralen Antidiabetika und Verfahren zu ihrer Herstellung.**

(30) Priorität: **08.06.83 DE 3320583**

(43) Veröffentlichungstag der Anmeldung:
**19.12.84 Patentblatt 84/51**

(45) Bekanntmachung des Hinweises auf die Patenterteilung:
**31.01.90 Patentblatt 90/5**

(84) Benannte Vertragsstaaten:
**AT BE CH DE FR IT LI LU NL SE**

(56) Entgegenhaltungen:
**EP-A- 0 068 191**
**EP-A- 0 086 468**
**DE-A- 2 355 743**
**DE-A- 3 100 535**
**DE-A- 3 225 188**

(73) Patentinhaber: **Dr. Karl Thomae GmbH, Postfach 1755, D-7950 Biberach (Riss) (DE)**

(72) Erfinder: **Brickl, Rolf, Dr.Dipl.-Chem., Erlenweg 37, D-7951 Warthausen (DE)**
Erfinder: **Schepky, Gottfried, Dr., Ulrich-von-Hutten-Weg 2, D-7950 Biberach 1 (DE)**
Erfinder: **Rupprecht, Eckhard, Dr.Dipl.-Biologe, Riedbachstrasse 15, D-7960 Aulendorf-Tannhausen (DE)**
Erfinder: **Greischel, Andreas, Dr., Dunantstrasse 5, D-7950 Biberach 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 128 482 B1

ACTORUM AG

## Beschreibung

Die Erfindung betrifft neue galenische Zubereitungsformen von oralen Antidiabetika und Verfahren zu ihrer Herstellung.

Die oralen Antidiabetika enthalten als Wirkstoffe Sulfonylharnstoffe, wie Gliquidon, oder substituierte Phenylcarbonsäuren. Als weitere Sulfonylharnstoffe kommen unter anderem bevorzugt in Betracht Glibenclamid, Glibornurid, Glisoxepid, Glipizid und Gliclazid. Bei Gliquidon handelt es sich um den

1-Cyclohexyl-3-[[p-[2-(3,4-dihydro-7-methoxy-4,4-dimethyl-1,3-dioxo-2-(1H)-isochinolyl)-ethyl]-phenyl]sulfonyl]harnstoff,

der eine blutzuckersenkende Wirkung ausübt. Als weitere antidiabetisch wirkende Substanzen kommen aber auch in Frage: 4-[2-(Aroylamino)ethyl]-benzoesäuren der allgemeinen Formel

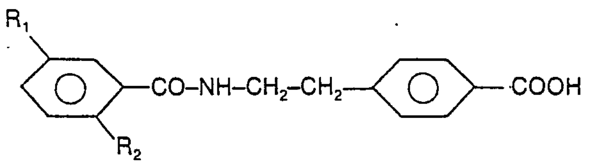

, (I)

worin $R_1$ ein Halogenatom, vorzugsweise ein Chloratom und $R_2$ eine Alkoxygruppe mit 1 bis 3 Kohlenstoffatomen, vorzugsweise die Methoxygruppe, oder eine Piperidin-1-yl- oder die Octamethyleniminogruppe bedeuten, desweiteren substituierte 4-(Aralkylaminocarbonylmethyl)-benzoesäuren der allgemeinen Formel

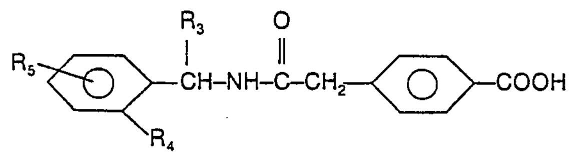

, (II)

worin $R_3$ eine Alkylgruppe mit 1 bis 4 Kohlenstoffatomen, vorzugsweise die n-Propylgruppe, oder die Phenylgruppe, $R_4$ die Piperidin-1-yl-, Pyrrolidin-1-yl- oder Hexamethyleniminogruppe und $R_5$ ein Wasserstoff- oder Halogenatom, vorzugsweise ein Chlor- oder Fluoratom, oder die Methyl- oder Methoxygruppe darstellen. Es können aber auch Gemische dieser Wirkstoffe verwendet werden.

Mikronisiertes Gliquidon ist in einem sich im Handel befindenden Präparat, bestehend ansonsten aus Maisstärke, Milchzucker und Magnesiumstearat, enthalten. Dieses gliquidonhaltige Präparat hat sich bereits als sicheres Antidiabetikum bewährt mit dem großen Vorteil, daß es bei eingeschränkter Nierenfunktion nicht kontraindiziert ist.

Im allgemeinen bestehen bei der oralen Applikation von in den Verdauungsflüssigkeiten schwerlöslichen Substanzen, und zu diesen zählen auch die oben genannten Substanzen, folgende Probleme: Der Wirkstoff kann in manchen Fällen nur unvollständig resorbiert werden und es können auch inter- und intraindividuell stark schwankende Blutspiegel des Wirkstoffes auftreten. Bei oralen Antidiabetika sind jedoch darüberhinaus der Wirkungseintritt und die Wirkungsdauer von besonderer Bedeutung, da die Wirkung auf die durch Nahrungsaufnahme hervorgerufenen Blutzuckerspiegel abgestimmt sein sollte. Dies ist mit den bisher zur Verfügung stehenden Zubereitungen von Antidiabetika, bei denen sich Substanzwirkung und physiologischer Insulinbedarf nach der Nahrungsaufnahme zeitlich nicht in zuverlässiger Weise aufeinander abstimmen lassen, nicht der Fall. Die Substanzwirkung setzt oft zu spät ein; oft wird die maximale Wirkung erst zu einem Zeitpunkt erreicht, zu welchem die Blutglukosewerte nach der Nahrungsaufnahme auch ohne Medikation bereits abfallen. Danach hält die Substanzwirkung noch an, wenn die Blutglukose bereits wieder den Ausgangswert erreicht (vgl. Berger, in Pelzer und Froesch, Diabetische Enteropathie, Hypoglykämien, Verlag Hans Hüber, Bern-Stuttgart-Wien 1974).

Es ist auch versucht worden, die blutzuckersenkende Wirkung eines Sulfonylharnstoffes mit dem nahrungsbedingten Anstieg des Blutzuckers zu synchronisieren, indem der Sulfonylharnstoff entsprechende Zeit vor der Mahlzeit eingenommen wurde. Dabei zeigte sich aber, daß eine Einnahme des Wirkstoffes 30 Minuten vor der Mahlzeit zu keiner befriedigenden Wirkungsverbesserung führte (vgl. Sartor et al., Eur. J. Clin. Pharmacolog. 21, 403 bis 408 (1982)), u. a. wegen der oben genannten längeren Wirkungsdauer. Eine bestimmte Zeitdifferenz zwischen Einnahme des Medikaments und der Nahrungsaufnahme läßt sich zudem nur in einer Klinik zuverlässig kontrollieren.

Man versuchte, diese Probleme bei Substanzen, die in den Verdauungsflüssigkeiten schwer löslich sind, dadurch zu lösen, daß man bestrebt war, bei der Entwicklung der galenischen Zubereitungen das Lösungsverhalten (Dissolution Rate) des für sich allein schwerlöslichen Wirkstoffes zu optimieren. Dies gelang beispielsweise durch eine Vergrößerung der Oberfläche des Wirkstoffes. So wird eine Zubereitungsform beschrieben (DE-PS-2 348 334), worin der Wirkstoff (auch ein blutzuckersenkender Stoff) mit einer Teilchenoberfläche von 3 bis 10 $m^2/g$ in Gegenwart eines Netzmittels vorliegt.

Dieses Ziel sollte aber auch dadurch erreicht werden, daß der Wirkstoff in gelöster Form auf einen Träger mit möglichst großer Oberfläche aufgezogen und das Lösungsmittel entfernt wird (vgl. H. Rupprecht, Acta Pharm. Technol. 26/1, Seite 13 u. f. (1980)).

Es wurde darüberhinaus versucht, die Dissolution Rate durch den Zusatz von Salzbildnern zu verbessern (vgl. DE-OS-3 124 090.9). Zur Verbesserung der Löslichkeit und der Auflösungsgeschwindigkeit wurden aber auch feste Dispersionen hergestellt. Sie bestehen aus dem Wirkstoff und einem oder mehreren wasserlöslichen Trägern, gegebenenfalls noch in Kombination mit oberflächenaktiven Stoffen. Zur Herstellung dieser Dispersionen wird eine homogene Schmelze aus dem Wirkstoff oder gegebenenfalls dessen Salz und einem Träger hergestellt (vgl. DE-OS-2 355 743). Bei einem anderen Verfahren werden Wirkstoff und Träger in einem gemeinsamen Lösungsmittel gelöst, anschließend wird das Lösungsmittel entfernt. Als wasserlösliche Träger werden u. a. Polyvinylpyrrolidon bzw. Polyethylenglykole verwendet (vgl. H. R. Merkle, Acta Pharm. Technol 27/4, S. 193 u. f. (1981) und W. L. Chiou, S. Riegelmann, J. Pharm. Scie. 60/9, 1281 u. f. (1971)).

Wendet man die nachstehenden, in der Literatur beschriebenen Verfahrensweisen für die Herstellung von die eingangs genannten Verbindungen enthaltenden Zubereitungsformen an, so erzielt man kaum eine bessere Dissolution Rate des Wirkstoffes, z. B. des Gliquidons: die Salzbildung für sich allein bringt keine Erhöhung der Dissolution Rate (vgl. Tabelle 6, Beispiel 1c), das Auftragen von Wirkstoff, z. B. von Gliquidon, auf einen Träger allein (vgl. Beispiel Seite 8) erbringt ebenfalls nicht den gewünschten Erfolg. Bei entsprechenden Versuchen, die später noch eingehend geschildert werden, wurde die Dissolution Rate bestimmt und im Falle von Gliquidon als nicht größer gefunden als die Auflösungsgeschwindigkeiten, die an sich bekannte gliquidonhaltige Zubereitungsformen zeigen.

Es wurde nun überraschenderweise gefunden, daß sich die die eingangs erwähnten Verbindungen enthaltenden insbesondere aber die gliquidonhaltigen Zubereitungsformen mit einer sehr raschen und vollständigen Freigabe des Wirkstoffes dadurch herstellen lassen, daß sauer oder amphoter reagierende Wirkstoffe mit Hilfe basischer Hilfsstoffe in Gegenwart eines oder mehrerer lösungsvermittelnder und/oder emulgierender Stoffe in eine Lösung übergeführt werden und die Lösung auf einen wasserunlöslichen Träger aufgebracht und getrocknet wird, und dieses Produkt gegebenenfalls noch unter Zusatz von Hilfsstoffen zu einer Arzneiform weiterverarbeitet wird. Gegenstand der Erfindung sind aber auch die so gewonnenen Zubereitungsformen selbst. In jedem Fall muß aber das molare Verhältnis von Wirkstoff zu basischem Hilfsstoff so gewählt werden, daß ein Überschuß an basischem Hilfsstoff vorliegt.

Wichtig ist, daß soviel an basischem Hilfsstoff dem Wirkstoff zugesetzt wird, daß in vivo eine rasche und vollständige Auflösung erfolgt. Dies ist erst bei einem molaren Verhältnis Wirkstoff zu basischem Hilfsstoff kleiner als 1 : 1 möglich.

Um beispielsweise 2,5 Gewichtsteile Gliquidon in 50 Gewichtsteilen Wasser in Lösung zu bringen, sind 0,7 Gewichtsteile Ethylendiamin x $1H_2O$, 3,0 Gewichtsteile N-Methylglucamin oder 3,5 Gewichtsteile Diethanolamin notwendig. Vergleicht man die molaren Verhältnisse, die unbedingt nötig sind für eine rasche und vollständige Auflösung des Wirkstoffes, so ergibt sich folgendes Bild:

Gliquidon (Mol.gew. 527,6: Ethylendiamin x $1H_2O$ (Mol.gew. 78,1) wie 1 : 1,89;
Gliquidon: N-Methylglucamin (Mol.gew. 195,21) wie 1 : 3,24;
Gliquidon: Diethanolamin (Mol.gew. 105,14) wie 1 : 7,03;
Gliquidon: L-Lysin (Mol.gew. 146,2) wie 1 : 4,33.

Dieser Befund läßt sich mit einer reinen Salzbildung des Gliquidons mit den jeweiligen basischen Hilfsstoffen nicht erklären; es scheint, daß die überschüssige Base einen zusätzlichen stabilisierenden Effekt ausübt. Dieselben Erscheinungen treffen auch für die übrigen Wirkstoffe zu. Dieser Effekt war auch für den Fachmann nicht vorhersehbar.

Als basische Hilfsstoffe eignen sich eine Reihe anorganischer oder organischer Basen, die mindestens in den angewandten Dosisbereichen physiologisch unbedenklich sind, beispielsweise Natronlauge, Kalilauge, Ammoniak, tert.Natriumphosphat, Diethanolamin, Ethylendiamin, N-Methylglucamin oder L-Lysin. Das molare Verhältnis von Wirkstoff zu basischem Hilfsstoff bzw. Hilfsstoffgemischen beträgt vorzugsweise 1 : 1,1 bis 1 : 10, ein darüberhinausgehender Überschuß an Base kann aber in manchen Fällen auch von Vorteil sein.

Um so hoch konzentrierte Lösungen, wie sie bei der Applikation eines erfindungsgemäßen Präparates offensichtlich erzeugt werden, zu stabilisieren, ist der Zusatz weiterer lösungsvermittelnder und/oder emulgierender Stoffe notwendig. Als solche Stoffe kommen u. a. Polyvinylpyrrolidone, Polyethylenglykol 4000 oder 6000, polyäthoxylierte Sorbitanmonooleate, Sorbit, Polyoxyethylenpolyoxypropylen-Polymere, Glycerin-Polyethylenglykoloxystearate und Polyoxyethylenfettalkoholether in Frage. Dabei spielen sowohl die Art des lösungsvermittelnden Stoffes als auch die jeweiligen Mengenverhältnisse eine wichtige Rolle für die Dissolution Rate des Wirkstoffes. Das Gewichtsverhältnis von Wirkstoff, z. B. Gliquidon, zur Gesamtmenge lösungsvermittelnder Stoffe beträgt vorzugsweise 1 : 1 bis 1 : 10.

Zur Herstellung der Lösung von Wirkstoff, basischen Hilfsstoffen sowie lösungsvermittelnden und/oder emulgierenden Stoffen dienen in erster Linie Wasser oder andere polare Lösungsmittel, wie niedere Alkohole, z. B. Ethanol, Isopropanol, Ketone, wie Azeton oder Gemische dieser Stoffe mit Wasser.

Die so hergestellten Lösungen werden auf wasserunlösliche Träger aufgebracht. Als solche eignen sich vorzugsweise Stoffe, die die Oberfläche vergrößern, wie hochdisperses Siliziumdioxid, Avicel® (mikrocristalline Cellulose), basische Aluminiumoxide, Magnesium-Aluminium-Trisilikate, quer vernetztes Polyvinylpyrrolidon, Natriumcarboxymethylstärke, Tricalciumphosphat, Calciumhydrogenphosphat und deren Gemische. Im allgemeinen kommt man mit einem Wirkstoff-Träger-Verhältnis von 1 : 1 bis 1 : 12 Gewichtsteilen aus. Als Trägerstoffe eignen sich ganz besonders solche, die sich in Wasser oder in einem anderen betreffenden Lösungsmittel nicht lösen; diese Trägerstoffe erlauben eine einfachere Handhabung sowohl bei der Einarbeitung des Wirkstoffes als auch bei der Weiterverarbeitung des Zwischenproduktes.

Durch die Verwendung der erfindungsgemäßen Lösungsmethode anstelle des aus der DE-OS-2 355 743 bekannten Schmelzverfahrens zur Einarbeitung des Wirkstoffes lassen sich auch nichtschmelzbare, lösungsvermittelnde Stoffe, wie das besonders günstig wirkende Polyvinylpyrrolidon, molekulardispers zusammen mit Gliquidon bzw. den anderen Wirkstoffen auf dem Träger verteilen. Im übrigen bieten aber wasserunlösliche Träger für die galenische Bearbeitung sehr große Vorteile.

Die vorstehend geschilderte Lösung des Problems ist aus den folgenden Gründen überraschend:

Die in der Literatur beschriebenen, nachstehend aufgeführten Verfahrensweisen für die Einarbeitung von in den Verdauungsflüssigkeiten schwer löslichen Substanzen bewirken, angewandt auf die Herstellung von beispielsweise gliquidonhaltigen Zubereitungsformen, keine deutliche Erhöhung der Dissolution Rate des Wirkstoffes, sie vermögen auch die für die im Handel sich befindenden gliquidonhaltigen Zubereitungsformen gefundenen Dissolution Rate nicht zu verbessern. Die Dissolution Rates wurden nach 5 bzw. 30 Minuten nach der USP XX Paddle Methode in 900 ml Mc Ilvaine-Puffer, pH-Wert 7,0, bei 37 °C und 1000 Umdrehungen pro Minute bestimmt. Je Bestimmung wurde eine 40,0 mg Wirkstoff entsprechende Menge an Zubereitungsform eingesetzt, jede Bestimmung wurde 2-mal wiederholt und der Mittelwert aus den jeweiligen Ergebnissen berechnet.

Zur Bestimmung der Dissolution Rate bei einer Oberflächenvergrößerung von Gliquidon wurden 30 Gewichtsteile des Wirkstoffes in 150 Gewichtsteilen Methylenchlorid gelöst und die Lösung auf 210 Gewichtsteile eines Tablettenträgers aufgezogen. Nach dem Trocknen wurde der beaufschlagte Tablettenträger zu Tabletten verpreßt und die Auflösungsgeschwindigkeit des Gliquidons aus diesen Tabletten bestimmt; nach 5 Minuten lösten sich 5%, nach 30 Minuten 7% des Wirkstoffes. Bei mikronisiertem Gliquidon ohne Hilfsstoffe waren nach 5 und nach 30 Minuten 0% gelöst. Verpresst man das mikronisierte Gliquidon zu Tabletten gemäß Beispiel 1b, so waren nach 5 Minuten 5,8% und nach 30 Minuten 7,2% Wirkstoff gelöst.

Auch die Salzbildung des Gliquidons ergab keine günstigeren Dissolution Rates. In einer wäßrigen Lösung von Ethylendiamin wurde Gliquidon unter Erwärmen und Rühren aufgelöst und, wie im Beispiel 1c beschrieben, weiterverarbeitet. Auch dieses Produkt ergab nach 5 Minuten und nach 30 Minuten eine Menge von jeweils nur 4% an gelöstem Wirkstoff.

Nichteinmal die Verwendung einer gliquidonhaltigen Dispersion zeigte bessere Dissolution Rates. Analog zu der in der DE-OS-2 355 743 beschriebenen Methode wurden 1,47 Gewichtsteile Gliquidon in einer Schmelze aus 79,1 Gewichtsteilen Polyethylenglykol 4000 und 5,0 Gewichtsteilen Polyoxyethylen-40-stearat gelöst und anschließend darin 14,43 Gewichtsteile Kaliumbikarbonat dispergiert. Die erstarrte Schmelze wurde durch ein Sieb der Maschenweite 1,0 mm gerieben. Die Bestimmung der Dissolution Rate ergab nach 5 Minuten 10% und nach 30 Minuten 7% an Wirkstoff.

In einer weiteren Versuchsreihe wurde geprüft, ob der Einsatz von Gliquidon-Salzen in das in der DE-OS-2 355 743 beschriebene Verfahren zu besseren Auflösungsraten führt. Verwendet wurde wiederum eine Schmelze aus 79,1 Gewichtsteilen Polyethylenglykol 4000 und 5,0 Gewichtsteilen Polyoxy-ethylen-40-stearat, in der eine gesättigte Lösung des betreffenden Gliquidon-Salzes hergestellt wurde. Anschließend wurden in dieser Lösung 14,43 Gew.T. Kaliumhydrogencarbonat dispergiert. Die erstarrte Schmelze wurde durch ein Sieb der Maschenweite 1,0 gerieben.

Tabelle

| Gliquidonsalz mit | In der Schmelze aus PEG 4000 und Polyoxyethylen-40-stearat waren maximal an Wirkstoff löslich (umgerechnet als Base): | Für 30 mg Gliquidon pro Dosis werden an fester Lösung benötigt: |
|---|---|---|
| Ethylendiamin | 0,65% | 4,6 g |
| $NH_4OH$ | 2,40% | 1,25 g |
| N-Methylglucamin | 0,54% | 5,54 g |
| Piperidin | 2,15% | 1,395 g |
| NaOH | 1,99 % | 1,51 g |

(PEG 4000 = Polyethylenglykol 4000)

Aus diesen Ergebnissen ist unschwer zu ersehen, daß die für 30 mg Gliquidon benötigte Menge an Schmelze in keiner schluckbaren und zerfallenden Tablette unterzubringen ist. Somit scheidet dieses Verfahren nach DE-OS-2 355 743 auch für Gliquidon-Salze aber auch für die Salze mit den anderen oben genannten Wirkstoffen aus.

Wie aus den vorstehend geschilderten Versuchen ersichtlich wird, läßt sich die Erzielung einer raschen und vollständigen Auflösung des Wirkstoffes, demonstriert am Gliquidon, bei Anwendung der bekannten, für solche Zwecke als geeignet beschriebenen Methoden nicht erreichen.

Werden nun die eingangs erwähnten blutzuckersenkenden Wirkstoffe nach den vorstehend beschriebenen Verfahren formuliert, so erhält man Arzneimittel, bei denen die Wirkung des Wirkstoffes auf den physiologischen Bedarf des Patienten an diesem Medikament abgestimmt ist. Diese besonderen Arzneimittel gewährleisten eine rasche und vollständige Resorption des Wirkstoffes. Eine rasche Resorption verkürzt die Zeit, die zwischen der Einnahme des Medikaments und der Einnahme der Mahlzeit liegen muß, um die blutzuckersenkende Wirkung des Sulfonylharnstoffes mit dem nahrungsbedingten Anstieg des Blutzuckers weitgehend zu synchronisieren. Eine rasche und vollständige Resorption verringert intra- und interindividuelle Streuungen des Blutglukose-Spiegels, sie minimiert die Abhängigkeit der Resorption vom Zustand des Magen-Darmtraktes bzw. von Art und Menge der aufgenommenen Nahrung und gewährleistet damit eine bedarfsgerechte Stoffwechselführung und damit verbunden eine bedarfsgerechte Insulinausschüttung. Die eingangs beschriebenen Nachteile der derzeitig bekannten Formen werden bei Anwendung des erfindungsgemäßen Verfahrens vermieden.

Im folgenden soll auf das Wesen der Erfindung noch näher eingegangen werden, indem die aus den Versuchen der Beispiele erzielten Ergebnisse besprochen werden. Die Versuche wurden vorwiegend mit dem Sulfonylharnstoff Gliquidon durchgeführt.

Das nachstehende Beispiel 1 beschreibt eine Zubereitungsform, bestehend aus Gliquidon, einem basischen Hilfsstoff und einem die Oberfläche vergrößernden Träger und zeigt, daß die Dissolution Rate der erfindungsgemäßen Gliquidonformulierung im Vergleich zu der Dissolution Rate von an sich bekannten Gliquidonformulierungen signifikant gesteigert wird.

Die bei einem Zusatz an lösungsvermittelnden Stoffen erzielten Verbesserungen in der Dissolution Rate gegenüber den Ergebnissen des Beispiels 1a schildern die tabellarisch wiedergegebenen Befunde der Beispiele 2 bis 9, die in der Tablelle 1 zusammengefaßt sind.

In der Tabelle 2 sind Beispiele mit gleichen Mengen an verschiedenen Trägern mit den dazugehörenden Dissolution Rates zusammengestellt. Da die Gewichtsverhältnisse Gliquidon zu Ethylendiamin als Base zu Kollidon 25® als Lösungsvermittler konstant gehalten wurden, ersieht man den Einfluß des Trägerstoffes auf die Dissolution Rate.

Der Einfluß der jeweiligen Menge an lösungsvermittelndem Hilfsstoff auf die Dissolution Rate wurde am Beispiel von Kollidon 25® in der Tabelle 3 sichtbar gemacht. Die Tabelle 3 zeigt auch, daß die Steigerung des lösungsvermittelnden Stoffes im Gegensatz zu einer Steigerung des Trägers (hochdisperses Siliciumdioxid, vgl. diesbezüglich mit Tabelle 1) zu keiner Verschlechterung sondern vielmehr zu einer Steigerung der Dissolution Rate führt.

Den Einfluß der jeweiligen Menge an wasserunlöslichen Trägern auf die Dissolution Rate faßt schließlich Tabelle 4 anhand weiterer Beispiele zusammen. Wie diese Tabelle zeigt, ist es von Vorteil, ein Zuviel an Trägerstoffen zu vermeiden.

Die Tabelle 5 zeigt, daß anstelle von Ethylendiamin auch andere alkalische, toxikologisch unbedenkliche Hilfsstoffe verwendet werden können.

Bei den Entwicklung von Arzneiformen erfolgt die Optimierung mit Hilfe von in vitro Methoden. Die Freisetzung und Auflösung des Wirkstoffes wird hierbei mit Hilfe von Dissolution Tests ermittelt. Um der in vivo Situation vergleichbare Verhältnisse zu schaffen, werden diese Tests normalerweise im Sauren bei pH 1,2 durchgeführt. Wendet man diesen pH bei den erfindungsgemäßen Formen an, so erhält man keine meßbaren Freisetzungsraten. In vitro Dissolution Tests müssen deshalb bei pH 7 (oder darüber) durchgeführt werden. Dies dürfte darauf zurückzuführen sein, daß die Löslichkeit des Wirkstoffs bei pH-Werten unter 7 nicht mehr ausreicht. Es mußte deshalb erwartet werden, daß auch in vivo im sauren Bereich des Intestinaltraktes nur eine relativ geringe Freisetzung des Wirkstoffes erfolgen würde. Die rasche und vollständige Resorption des Wirkstoffs auch im oberen Bereich des Intestinaltraktes ist deshalb auch für den Fachmann überraschend. Weiterhin ist überraschend, daß trotz der Unterschiede zwischen in vivo Situation und der Bestimmung der Dissolution Rate in vitro eine weitgehende Übereinstimmung zwischen in vitro und in vivo besteht. Dies zeigt sich in den Beispielen 1c, 17 und 22, wenn man deren Ergebnisse, die in Tabelle 6 zusammengestellt sind, mit den entsprechenden Human-Blutzuckersenkungskurven, die in den Abbildungen 6 und 7 aufgezeichnet sind, vergleicht.

Es folgen die diesen Betrachtungen zugrundeliegenden Beispiele mit genauen Zahlenangaben:

In den Beispielen ist:
Avicel® eine mikrokristalline Cellulose,
Kollidon 25® ein Poly-N-vinylpyrrolidon(-2),
Tween 80® ein Polyoxyethylen(20)sorbitanmonooleat,
Pluronic F 68® ein Polyoxyethylenpolyoxypropylen-Polymer,
Cremophor RH 40® ein Glycerin-Polyethylenglykoloxystearat,
Aerosil® ein röntgenamorphes Siliciumdioxid,

Masigel® ein Di-Magnesium-Aluminium-Trisilikat,
Explotab® die Natriumcarboxymethylstärke und
Kollidon CL®, ein vernetztes, unlösliches Polyvinylpyrrolidon
Amberlite IRP 88, Kaliumsalz von Polymeren aus Methacrylsäure und Divinylbenzol (Methacrylic acid polymer with divinylbenzene, potassium salt).

Beispiel 1:

a) Zusammensetzung eines gliquidonhaltigen Pulvers:

| | | |
|---|---|---|
| 5 | Gewichtsteile Gliquidon | (1) |
| 1,9 | Gewichtsteile Ethylendiamin x $H_2O$ | (2) |
| 20 | Gewichtsteile Avicel® | (3) |

Herstellung:

Das basische Hilfsmittel (2) wird in 100 Gewichtsteilen Wasser von 70 °C unter Rühren gelöst. Der Wirkstoff (1) wird zugegeben; man rührt, bis dieser vollständig gelöst ist. In diese Lösung wird der Träger (3) suspendiert. Die Suspension wird im Vakuum unter Rühren zur Trockene eingedampft und das Produkt durch ein Sieb von 1 mm Maschenweite gerieben.

Gefundene Dissolution Rate:
nach 5 Minuten waren 31% Gliquidon,
nach 30 Minuten waren 53% Gliquidon gelöst.

b) Vergleich mit einem bekannten gliquidonhaltigen Präparat folgender Zusammensetzung:

| | |
|---|---|
| mikronisiertes Gliquidon | 30 Gewichtsteile |
| Maisstärke | 75 Gewichtsteile |
| Milchzucker | 132 Gewichtsteile |
| Magnesiumstearat | 3 Gewichtsteile |

Gefundene Dissolution Rate:
nach 5 Minuten: 5,8%
nach 30 Minuten: 7,2%

c) Vergleich mit einem gliquidonhaltigen Pulver ohne Trägerstoff:

| | | |
|---|---|---|
| 5 | Gewichtsteile Gliquidon | (1) |
| 1,9 | Gewichtsteile Ethylendiamin x $H_2O$ | (2) |

Herstellung:

Das basische Hilfsmittel (2) wird in 100 Gewichtsteilen Wasser von 70 °C unter Rühren gelöst. Der Wirkstoff (1) wird zugegeben; man rührt, bis dieser vollständig gelöst ist. Diese Lösung wird im Rotationsverdampfer unter Vakuum getrocknet und das feste Produkt durch ein Sieb der Maschenweite 1,00 mm gesiebt.

Gefundene Dissolution Rate:
nach 5 Minuten: 4%;
nach 30 Minuten: 4%.

Beispiele 2 bis 9:

Die in nachfolgender Tabelle 1 zusammengefaßten Beispiele 2 bis 9 zeigen die Möglichkeiten zur Beeinflussung der Dissolution Rate (in % gelöstem Gliquidon) bei Verwendung verschiedener, lösungsvermittelnder Substanzen sowie bei quantitativen Änderungen ein- und desselben Trägerstoffes. Die Herstellung der verschiedenen Rezepturen wurde wie folgt durchgeführt:

Der basische Hilfsstoff wurde in Wasser von 70 °C unter Rühren gelöst, das Gliquidon wurde zugegeben. Man rührte bei 70 bis 80 °C solange, bis der Wirkstoff gelöst war. Daraufhin wurden die restlichen Bestandteile eingerührt und die Suspension im Vakuum eingedampft. Das so entstandene Produkt ließ man durch ein Sieb der Maschenweite 1 mm passieren.

Tabelle 1

| Beispiel Nr. | Gliquidon mg | Ethylendiamin x $H_2O$ als basischer Hilfsstoff mg | lösungsvermittelnder Hilfsstoff Menge mg | Art | als Träger kolloidale Kieselsäure mg | Dissolution Rate als Prozent des in Lösung gegangenen Wirkstoffes nach 5 | und 30 Minuten |
|---|---|---|---|---|---|---|---|
| 2 | 5,0 | 1,9 | 30 | Kollidon 25® | 60 | 65 | 83 |
| 3 | 5,0 | 1,9 | 30 | Polyethylenglykol 6000 | 60 | 39 | 59 |
| 4 | 5,0 | 1,9 | 30 | Tween 80® | 60 | 34 | 40 |
| 5 | 5,0 | 1,9 | 30 | Sorbitol | 60 | 29 | 30 |
| 6 | 5,0 | 1,9 | 30 | Pluronic F68® | 60 | 54 | 60 |
| 7 | 5,0 | 1,9 | 30 | Kollidon 25® + 33,1 mg Cremophor RH 40® | 30 | 79 | 89 |
| 9 | 5,0 | 1,9 | 30 | Kollidon 25® + 33,1 mg Cremophor RH 40® | 15 | 91 | 96 |

Beispiele 10 bis 14:

In der Tabelle 2 sind die Beispiele 10 bis 14, aber auch das Beispiel 2 zusammengefaßt. Es handelt sich um Beispiele von Zubereitungsformen mit gleichen Mengen an verschiedenen Trägern, gleichen Mengen an gleichem basischem Hilfsstoff, an Gliquidon und an gleichem lösungsvermittelnden Hilfsstoff. Die Tabelle 2 zeigt den Einfluß des jeweiligen Trägerhilfsstoffes auf die Dissolution Rate. Die Herstellung der einzelnen Rezepturen erfolgte wie für Tabelle 1 beschrieben.

Tabelle 2
Die Beispiele 2 und 10 bis 14 zeigen folgende Zusammensetzungen:
5 mg Gliquidon, 1,9 mg Ethylendiamin x $H_2O$; 30 mg Kollidon 25®

| Beispiel | Trägerstoff 60 mg<br>Art | Dissolution Rate in Prozent des in Lösung gegangenen Wirkstoffes nach<br>5 | und 30 Minuten |
|---|---|---|---|
| 2 | Aeorosil ® | 65 | 83 |
| 10 | Avicel® | 87 | 92 |
| 11 | basisches Aluminiumoxid | 56 | 64 |
| 12 | Masigel® | 80 | 91 |
| 13 | Kollidon CL® | 89 | 90 |
| 14 | Explotab® | 95 | 95 |

Beispiele 15 und 16:

Die nachfolgende Tabelle 3 enthält die Beispiele 15 und 16 zusammen mit dem Beispiel 2 zum Vergleich. Es handelt sich hierbei um Beispiele mit gleichen Mengen an Gliquidon, Ethylendiamin, Aerosil® aber unterschiedlichen jeweiligen Mengen an Kollidon 25®. Es wird der Einfluß der jeweiligen Menge an lösungsvermittelndem Hilfsstoff auf die in Dissolution Rate des Wirkstoffes dargestellt. Die Herstellung der einzelnen Rezepturen erfolgt wie für Tabelle 1 beschrieben.

Tabelle 3
Die Beispiele 2, 15 und 16 zeigen gemeinsam folgende Zusammensetzungen:
5 mg Gliquidon, 1,9 mg Ethylendiamin x $H_2O$, 60 mg Aerosil®

| Beispiel | Lösungsvermittelnder Stoff Kollidon 25®<br>mg | Dissolution Rate in Prozent des in Lösung gegangenen Wirkstoffes nach<br>5 | und 30 Minuten |
|---|---|---|---|
| 15 | 10 | 35 | 56 |
| 2 | 30 | 65 | 83 |
| 16 | 60 | 72 | 85 |

Beispiel 17

Das Beispiel 17 zeigt zusammen mit den Beispielen 2, 8, 9 und 10 den Einfluß der jeweiligen Menge an Trägerstoff auf die Dissolution Rate, die Werte sind der nachfolgenden Tabelle 4 zu entnehmen. Auch diese Rezepturen wurden analog denen in Tabelle 1 zubereitet. Der Tabelle 4 kann entnommen werden, daß größer werdende Mengen an Trägerstoffen die Dissolution Rate wieder reduzieren.

Tabelle 4
Die Beispiele 2, 8, 9, 10 und 17 zeigen gemeinsam folgende Zusammensetzungen:
5 mg Gliquidon, 1,9 mg Ethylendiamin x $H_2O$

| Beispiel | Lösungsvermittelnder Stoff Menge in mg | Art | Träger Menge in mg | Art | Dissolution Rate in Prozent des in Lösung gegangenen Wirkstoffes nach 5 | und 30 Minuten |
|---|---|---|---|---|---|---|
| 9 | 30 | Kollidon 25® und 33,1 mg Cremophor RH 40® | 15 | Aerosil ® | 91 | 96 |
| 8 | 30 | Kollidon 25® | 30 | Aerosil® | 79 | 89 |
| 2 | 30 | Kollidon 25® | 60 | Aerosil® | 65 | 83 |
| 17 | 30 | Kollidon 25® | 20 | Avicel® | 91 | 93 |
| 10 | 30 | Kollidon 25® | 60 | Avicel® | 87 | 92 |

Beispiele 18 bis 20

Den Einfluß unterschiedlicher basischer Hilfsstoffe auf die Dissolution Rate zeigen die in der Tabelle 5 aufgeführten Beispiele 18 bis 20. Die Herstellung der Rezepturen erfolgte gemäß Tabelle 1.

Tabelle 5
Die Beispiele 18 bis 20 zeigen gemeinsam folgende Zusammensetzungen:
5 mg Gliquidon, 27,5 mg Kollidon 25®.

| Beispiel | Basischer Hilfsstoff Menge in mg | Art | Träger Aerosil® mg | Dissolution Rate in Prozent des in Lösung gegangenen Wirkstoffes nach 5 | und 30 Minuten |
|---|---|---|---|---|---|
| 18 | 6 | N-Methylglucamin | 61,5 | 56 | 73 |
| 19 | 2,5 | Ethylendiamin x $H_2O$ | 65 | 62 | 75 |
| 20 | 0,48 | Natriumhydroxid | 67,2 | 62 | 82 |

Beispiele 21 und 22

Die Zubereitungsform des Beispiels 21 wurde wie für Tabelle 1 beschrieben hergestellt. Die Herstellung der Zubereitungsform des Beispiels 22 erfolgte durch gemeinsames Lösen des Wirkstoffes und des lösungsvermittelnden Stoffes in Ethanol; die Lösung wurde zur Trockne eingedampft und das Produkt durch ein Sieb der Maschenweite 1 mm gerieben.

Wie sich aus der Tabelle 6 ergibt, führt das alleinige Vorhandensein eines basischen Hilfsstoffes zu keiner brauchbaren Dissolution Rate (vgl. Beispiel 1c), auch das alleinige Vorhandensein eines lösungsvermittelnden Stoffes (es fehlt der basische Hilfsstoff und der Trägerstoff) führt zu keinem Produkt mit brauchbarer Dissolution Rate (vgl. Beispiel 22). Fügt man jedoch einen Trägerstoff hinzu (vgl. Beispiel 1a), so ist nach 30 Minuten über die Hälfte des Wirkstoffes in Lösung gegangen. Ist dann noch ein Lösungsvermittler beigemischt (vgl. Beispiel 17), so ergeben sich innerhalb sehr kurzer Zeit ausgezeichnete Werte. Hieraus wird ersichtlich, daß die Kombination Gliquidon mit basischem Hilfsstoff und Lösungsvermittler, in Gegenwart eines wasserunlöslichen Trägerstoffes, die besten Resultate bezüglich der raschen und möglichst vollständigen Auflösung des Wirkstoffes erbringt.

Tabelle 6

| Beispiel | Gliquidon mg | Basischer Hilfsstoff Ethylendiamin x $H_2O$ mg | 30 mg Lösungsvermittler Kollidon 25® | Träger mg | Art | Dissolution Rate in Prozent des in Lösung gegangenen Wirkstoffes nach 5 | und 30 Minuten |
|---|---|---|---|---|---|---|---|
| 17 | 5 | 1,9 | ja | 20 | Avicel® | 91 | 93 |
| 1c | 5 | 1,9 | nein | – | – | 4 | 4 |
| 21 | 25 | 6,5 | ja | 40 | Aerosil® | 49 | 65 |
| 22 | 25 | – | ja | – | – | 12 | 24 |
| 1a | 5 | 1,9 | nein | 20 | Avicel® | 31 | 53 |

Daß die gute Auflösungsgeschwindigkeit der erfindungsgemäßen Rezepturen nicht allein durch Salzbildung erklärbar ist, zeigen auch folgende Tablettenbeispiele, die in vitro und teilweise auch in vivo (vgl. Abbildungen 8 und 9) überprüft wurden:

Beispiel 23
a) 1 Tablette enthält:
38,31 mg Gliquidon · L-Lysin (Salz)
(= 30 mg Gliquidon-Base)
130,0 mg Explotab
130,0 mg Avicel
1,69 mg Magnesiumstearat
300,0 mg
Aus der Mischung Explotab, Avicel und Magnesiumstearat werden runde bikonvexe Tabletten von 300 mg und 10 mm Durchmesser gepreßt und mit Hydroxypropylmethylcellulose geschmacksabdeckend überzogen.
Dissolution rate: nach 5 Minuten: 0%
nach 30 Minuten: 11%

b) Wirkstoffgranulat/Tablette
30,0 mg Gliquidon
9,0 mg L-Lysin
24,0 mg Kollidon
48,0 mg Explotab
Die Verarbeitung erfolgt analog den Beispielen 2 bis 9.
Pro Tablette werden hinzugefügt:
94,0 mg Explotab
94,0 mg Mikrokristalline Cellulose
1,0 mg Magnesiumstearat
300,0 mg
Explotab, Cellulose und Magnesiumstearat werden zugemischt. Aus dieser Mischung werden runde, bikonvexe Tabletten von 300 mg und 10 mm Durchmesser gepreßt und mit Hydroxypropylmethylcellulose geschmacksabdeckend überzogen.
Dissolution rate: nach 5 Minuten: 46,3%
nach 30 Minuten: 51,2%

c) Wirkstoffgranulat/Tablette
30,0 mg Gliquidon
36,0 mg L-Lysin
20,0 mg Kollidon 25
24,0 mg Pluronid F 68
48,0 mg Avicel
Verarbeitung erfolgt analog den Beispielen 2 bis 9.
Pro Tablette werden hinzugemischt:
70,0 mg Avicel
70,0 mg Explotab
2,0 mg Magnesiumstearat
300,0 mg
Aus dieser Mischung werden runde, bikonvexe Tabletten von 300 mg und 10 mm Durchmesser gepreßt und geschmacksabdeckend mit Hydroxypropylmethylcellulose überzogen.
Dissolution rate: nach 5 Minuten: 100%
Wird in obiger Rezeptur der Salzbildner Lysin weggelassen und Gliquidon in der Lösung von Kollidon 25 und Pluronic F 68 in Wasser gelöst und anschließend das daraus resultierende Wirkstoffgranulat in gleicher Weise zu Filmtabletten verarbeitet, dann zeigen diese Tabletten folgende Dissolution rates:
nach 5 Minuten: 7,4%
nach 30 Minuten: 8,3%

Beispiel 24 – Filmtabletten
a) Wirkstoffgranulat/Tablette
30,0 mg Gliquidon
1,6 mg NaOH
20,0 mg N-Methylglucamin
20,0 mg Kollidon 25
24,0 mg Pluronic F 68
48,0 mg Avicel
Die Herstellung erfolgte analog den Beispielen 2 bis 9.
Dem Wirkstoffgranulat werden pro Tablette zugemischt:
77,0 mg Explotab
77,0 mg Avicel
2,4 mg Magnesiumstearat
300,0 mg
und die fertige Mischung zu runden, bikonvexen Tabletten von 300 mg und 10 mm Durchmesser verpreßt. Diese werden mit Hydroxypropylmethylcellulose geschmacksabdeckend überzogen.
Dissolution rate: nach 5 Minuten: 97,6%

b) Wirkstoffgranulat/Tablette
30,0 mg Gliquidon
36,0 mg N-Methylglucamin
20,0 mg Kollidon 25
24,0 mg Pluronic F 68
48,0 mg Avicel
Die Herstellung erfolgte analog den Beispielen 2 bis 9.
Dem Wirkstoffgranulat werden pro Tablette zugemischt:
70,0 mg Explotab
70,0 mg Avicel
2,0 mg Magnesiumstearat
300,0 mg
und die fertige Mischung zu runden, bikonvexen Tabletten von 300 mg und 10 mm Durchmesser verpreßt. Diese werden mit Hydroxypropylmethylcellulose geschmacksabdeckend überzogen.
Dissolution rate: nach 5 Minuten: 91,1%
nach 30 Minuten: 87,9%

Beispiel 25 – Filmtabletten
Wirkstoffgranulat/Tablette
30,0 mg Gliquidon
20,0 mg L-Lysin
1,6 mg NaOH
20,0 mg Kollidon 25
24,0 mg Pluronic F 68
48,0 mg Avicel
Die Herstellung des Wirkstoffgranulates erfolgte analog den Beispielen 2 bis 9.
Dem Wirkstoffgranulat werden pro Tablette zugemischt:

77,5 mg Explotab
77,5 mg Avicel
1,4 mg Magnesiumstearat
300,0 mg

und die fertige Mischung zu runden, bikonvexen Tabletten von 300 mg und 10 mm Durchmesser verpreßt. Diese werden mit einem geschmacksabdeckenden Hydroxypropylmethylcellulose-Überzug dragiert.

Dissolution rate: nach 5 Minuten: 93,6%

Beispiel 26 – Filmtabletten

1 Tablette enthält:

30 mg 4-[(1-(2-Piperidino-phenyl)-1-butyl)-aminocarbonylmethyl]-benzoesäure
134 mg Amberlite IRP 88
134 mg Avicel
2 mg Magnesiumstearat
300 mg

Die Tablettenbestandteile werden miteinander vermischt, zu runden, bikonvexen Tabletten von 300 mg und 10 mm Durchmesser verpreßt und mit Hydroxypropylmethylcellulose geschmacksabdeckend überzogen.

Dissolution rate: nach 5 Minuten: 25,6%
nach 30 Minuten: 36,3%

Beispiel 27 – Filmtabletten

Wirkstoffgranulat/Tablette

30 mg 4-[(1-(2-Piperidino-phenyl)-1-butyl)-aminocarbonylmethyl]-benzoesäure
36 mg L-Lysin
20 mg Kollidon 25
24 mg Pluronic F 68
48 mg Avicel

Die Verarbeitung erfolgt analog den Beispielen 2 bis 9.

Pro Tablette werden dem so hergestellten Granulat hinzugefügt:

70,5 mg Avicel
70,5 mg Amberlite IRP 88
1,0 mg Magnesiumstearat
300,0 mg

Aus dieser Mischung werden runde, bikonvexe Tabletten von 300 mg und 10 mm Durchmesser gepreßt und mit Hydroxypropylmethylcellulose geschmacksabdeckend überzogen.

Dissolution rate: nach 5 Minuten: 46,8%
nach 30 Minuten: 94,5%

Beispiel 28 – Filmtabletten

1 Tablette enthält:

30 mg 4-[N-(α-Phenyl-2-piperidino-benzyl)-aminocarbonylmethyl]-benzoesäure
134 mg Amberlite IRP 88
134 mg Avicel
2 mg Magnesiumstearat
300 mg

Die Tablettenbestandteile werden miteinander vermischt, zu runden, bikonvexen Tabletten von 300 mg und 10 mm Durchmesser verpreßt und mit Hydroxypropylmethylcellulose geschmacksabdeckend überzogen.

Dissolution rate: nach 5 Minuten: 15,8%
nach 30 Minuten: 20,9%

Beispiel 29 – Filmtabletten

Wirkstoffgranulat/Tablette

30 mg 4-[N-(α-Phenyl-2-piperidino-benzyl)-aminocarbonylmethyl]-benzoesäure
30 mg L-Lysin
20 mg Kollidon 25
24 mg Pluronic F 68
48 mg Avicel

Die Verarbeitung erfolgte analog den Beispielen 2 bis 9.

Pro Tablette werden dem so hergestellten Granulat hinzugefügt:

73,5 mg Avicel
73,5 mg Amberlite IRP 88
1,0 mg Magnesiumstearat
300,0 mg

Aus dieser Mischung werden runde, bikonvexe Tabletten von 300 mg und 10 mm Durchmesser gepreßt und mit Hydroxypropylmethylcellulose geschmacksabdeckend überzogen.

Dissolution rate: nach 5 Minuten: 53,6%
nach 30 Minuten: 98,2%

Beispiel 30 – Filmtabletten

1 Tablette enthält:

30 mg 4-[2-(5-Chlor-2-octamethylenimino-benzoylamino)-ethyl]-benzoesäure
134 mg Amberlite IRP 88
134 mg Avicel
2 mg Magnesiumstearat
300 mg

Die Tablettenbestandteile werden miteinander vermischt, zu runden, bikonvexen Tabletten von 300 mg und 10 mm Durchmesser verpreßt und mit Hydroxypropylmethylcellulose geschmacksabdeckend überzogen.

Dissolution rate: nach 5 Minuten: 18,4%
nach 30 Minuten: 27,2%

Beispiel 31 – Filmtabletten

Wirkstoffgranulat/Tablette

30 mg 4-[2-(5-Chlor-2-octamethylenimino-benzoylamino)-ethyl]-benzoesäure
36 mg L-Lysin
20 mg Kollidon 25
24 mg Pluronic F 68
48 mg Avicel

Die Verarbeitung erfolgt analog den Beispielen 2 bis 9.

Pro Tablette werden dem so hergestellten Granulat hinzugefügt:

70,5 mg Avicel
70,5 mg Amberlite IRP 88
1,0 mg Magnesiumstearat
300,0 mg

Aus dieser Mischung werden runde, bikonvexe Tabletten von 300 mg und 10 mm Durchmesser gepreßt und mit Hydroxypropylmethylcellulose geschmacksabdeckend überzogen.

Dissolution rate: nach 5 Minuten: 98,2%
nach 30 Minuten: 98,7%

Es folgen nach weitere Beispiele für die Herstellung pharmazeutischer Zubereitungsformen.

Beispiel 32
Kapseln

Eine 15 mg Gliquidon entsprechende Menge von Granulat aus Beispiel Nr. 9 wird nach Zumischen von einer entsprechenden Menge an Maisstärke und Magnesiumstearat in Hartgelatine-Kapseln der Größe 2 abgefüllt.

Beispiel 33
Kapseln

Eine 15 mg Gliquidon entsprechende Menge von Granulat aus Beispiel Nr. 13 wird nach Zumischen einer entsprechenden Menge an Maisstärke und Magnesiumstearat in Hartgelatine-Kapseln der Größe 1 abefüllt.

Beispiel 34
Filmtabletten

Eine 30 mg Gliquidon entsprechende Menge von Granulat des Beispiels Nr. 14 wird nach Zumischen von Avicel® und Magnesiumstearat zu ovalen Kernen mit 16 × 8 mm Durchmesser und 700 mg Gewicht auf einer Tablettiermaschine verpreßt. Anschließend werden die Kerne in einem Dragierkessel mit einem 14 mg Trockensubstanz entsprechenden, geschmacksabdeckenden Hydroxypropylmethylcellulose-Überzug versehen.

Beispiel 35
Filmtabletten

Eine 30 mg Gliquidon entsprechende Menge von Granulat des Beispiels Nr. 17 wird nach Zumischen eines Hilfsgranulates aus Milchzucker, Maisstärke und Aerosil® sowie nach Zugabe von Magnesiumstearat zu bikonvexen, runden Kernen von 11 mm Durchmesser und 400 mg Gewicht auf einer Tablettiermaschine verpreßt. Diese Kerne werden anschließend mit einem 8 mg Trockensubstanz entsprechenden, geschmacksabdeckenden Überzug von Hydroxypropylmethylcellulose in einem Dragierkessel überzogen.

Beispiel 36

3,5 mg Glibenclamid; 4,2 mg N-Methylglucamin; 19,25 mg Kollidon 25®; 14,0 mg Avicel werden analog wie für Tabelle 1 beschrieben verarbeitet. Von dieser Zubereitung wurde je Bestimmung eine 42 mg Glibenclamid entsprechende Menge eingesetzt.

Gefundene Dissolution Rate:
nach 5 Minuten: 97,9 ± 6,3% Glibenclamid
nach 30 Minuten: 93,6% ± 5% Glibenclamid.

Demgegenüber zeigte das entsprechende Handelsprodukt Euglucon N im Dissolution Test (12 Tabletten/900 ml):
nach 5 Minuten 16,8 ±1,9% Glibenclamid
nach 30 Minuten 13,8 ± 1,4% Glibenclamid.

Setzt man anstelle von 42 mg Glibenclamid je Bestimmung nur 21 mg Glibenclamid ein, so ergeben sich folgende Dissolution Rates:

a) bei obiger Zubereitung:
nach 5 Minuten 95,6 ± 4,4% Glibenclamid
nach 30 Minuten 98,8 ±0,7% Glibenclamid

b) bei Euglucon N (= 6 Tabletten/900 ml):
nach 5 Minuten 29,4 ± 2,3% Glibenclamid
nach 30 Minuten 27,3 ± 1,4% Glibenclamid.

Die erfindungsgemäße Zubereitung von Glibenclamid ist der Handelsform Euglucon N in der Dissolution Rate weit überlegen, was vor allem aus dem Vergleich hervorgeht, der mit 42 mg Glibenclamid/900 ml Flüssigkeit durchgeführt wurde.

Es folgen nun die Ergebnisse und Erläuterungen von Humanuntersuchungen, in denen gezeigt wird, daß die erfindungsgemäß hergestellten Arzneiformen die eingangs erwähnten Vorteile aufweisen.

Die Abbildung 1 zeigt den Verlauf der Blutzuckerspiegel nach der Gabe eines Präparates gemäß Beispiel 1b (Gliquidon-Handelsform), eines erfindungsgemäß hergestellten Präparates und eines Glibenclamid enthaltenden Präparates, mit dem Handelsnamen Euglucon N® bzw. Semi-Euglucon N®. Auf Grund der in der DE-PS-2 348 334 beschriebenen Humanuntersuchungen sowie den Literaturdaten von Euglucon N® muß angenommen werden, daß dieses Präparat analog der Rezeptur dieser Patentschrift hergestellt wird. Es zeigt sich, daß der Wirkungseintritt bei Applikation des erfindungsgemäßen Präparates wesentlich rascher einsetzt und kürzer anhält als bei den anderen beiden Präparaten. (Bei Anwendung des bekannten und gut wirksamen Glibenclamids wird erst ca. 1,5 Stunden nach der Applikation das Wirkungsmaximum erreicht, wobei die Wirkung erst nach mehr als 4 Stunden zu Ende geht.)

Die Abbildung 2 zeigt den Verlauf der Gliquidon-Plasmaspiegel nach der Gabe eines Präparates gemäß Beispiel 1b (Gliquidon-Handelsform) und eines erfindungsgemäß hergestellten Präparates. Es zeigt sich, daß der schnellere Wirkungseintritt eindeutig durch die schnellere Resorption hervorgerufen wird.

Die Abbildung 3 veranschaulicht den Verlauf der Blutzuckerspiegel nach der Gabe von Placebo und eines erfindungsgemäßen Präparates im Cross-Over an gesunden Personen bei einer Belastung mit 50 g Kohlehydrat in Form eines Gemisches aus Glukose, Di- und Oligosacchariden (Dextro OGT®). Die Abbildung 4 zeigt wiederum den Verlauf der Blutzuckerspiegel, jedoch bei Belastung mit einem Standardfrühstück. Das Standardfrühstück setzt sich zusammen aus 2 Tassen Schwarzen Tee mit jeweils 10 g Zucker, 2 halbe Brötchen mit jeweils 5 g Butter und 7 g Bienenhonig. Aus den Abbildungen 3 und 4 wird ersichtlich, daß der Blutzuckerspiegelanstieg nach der Kohlenhydratbelastung praktisch vollständig abgefangen wird. Dies bedeutet, daß der Wirkungseintritt bzw. Wirkungsverlauf dem Verlauf der

Kohlenhydratspiegel bei Nahrungsaufnahme optimal angepaßt ist.

Die Abbildung 5 zeigt den Verlauf des Blutzuckerspiegels bei Belastung mit einem Standardfrühstück nach der Gabe eines Präparates gemäß Beispiel 17 (erfindungsgemäßes Präparat), Beispiel 1b (Gliquidon-Handelsform), und zweier Präparate, die Glibenclamid enthalten (Euglucon N® und Semieuglucon N®), wobei das Frühstück 15 Minuten nach Arzneimittelgabe eingenommen wurde. Es zeigt sich bei einem der Präparate, daß eine höhere Dosierung zwar den Anstieg des Blutzuckerspiegels vermindert, dann aber einen starken Blutzuckerabfall nach einiger Zeit bewirkt. Dies bedeutet, daß auch mit einer Erhöhung der Dosis der gewünschte Effekt nicht ohne Risiko erreicht wird. Im Gegensatz zu den Handelsformen kann bei dem erfindungsgemäßen Präparat der Anstieg des Blutzuckerspiegels nach Frühstücksgabe praktisch vollständig abgefangen werden, ohne daß sich zu niedrige Blutspiegelwerte einstellen.

Die Abbildung 6 zeigt den Verlauf der Blutzuckerspiegel an einem Typ-2-Diabetiker nach der Gabe von Placebo und eines erfindungsgemäßen Präparates, wobei das Frühstück 10 Minuten nach Arzneimittelgabe eingenommen wurde. Wie man aus diesen Kurven ersieht, wird durch die Applikation des erfindungsgemäßen Präparates der Blutzuckeranstieg in Bezug auf Höhe und Dauer physiologischen Verhältnissen, wie sie bei Stoffwechselgesunden auftreten, angepaßt (vgl. Placebo-Kurve Abb. 4).

Die Abbildung 7 zeigt, wie auf Seiten 12 und 23 bereits beschrieben, die Übereinstimmung zwischen in-vitro- und in-vivo-Ergebnissen.

Aber auch die Messung der peripheren Insulinspiegel veranschaulicht den vorteilhaften Wirkungsverlauf der erfindungsgemäßen Präparate.

Aus der Messung der peripheren Insulinspiegel während Glukosebelastung bzw. Frühstück wurde die Menge an ausgeschüttetem Insulin berechnet. Durch das erfindungsgemäße Präparat wird insgesamt nicht mehr Insulin freigesetzt als durch die Glucose allein. Bei Berechnung von Teilflächen wird allerdings die frühe Stimulierung der mit dem erfindungsgemäßen Präparat behandelten Gruppe deutlich. In der Zeit von 0–42 Minuten wird im Vergleich zur Placebogruppe die doppelte Menge an Insulin freigesetzt (Tabelle 7). Für die Phase von 42–300 Minuten sind die Unterschiede gering und erreichen in keinem Fall die Signifikanzgrenze. Der starke Effekt auf den Blutzucker ist danach mit einer Verstärkung der frühen Insulinsekretion zu erklären. Eine therapeutisch unerwünschte zu langanhaltende Stimulierung der Insulinsekretion erfolgt nicht.

Tabelle 7
Teilflächen unter der Plasmainsulinkurve
Mittelwert ± SEM (µE min/ml)

| | | 0–42 Minuten | 42–300 Minuten |
|---|---|---|---|
| Placebo | Frühstück n = 6 | 722 ± 171 | 6260 ± 1346 |
| | Glukose n = 6 | 558 ± 156 | 4597 ± 900 |
| Erfind. gem. Präparat | Frühstück n = 6 | 1384 ± 298 | 5196 ± 873 |
| | Glukose n = 6 | 1486 ±312 | 5420 ± 810 |

Die Wirkung auf die Insulinsekretion von 7 Diabetikern, von denen 2 mit Diät allein eingestellt waren, 2 mit Euglucon® bzw. Semieuglucon N® (Glibenclamid-enthaltende Präparate) und 3 mit einem Gliquidon enthaltenden erfindungsgemäßen Präparat zeigte die Überlegenheit des neuen erfindungsgemäßen Präparates verglichen mit der jeweiligen Standardmedikation (Tabelle 8), die im cross-over geprüft wurde. Bezogen auf die basale Insulinsekretion zeigt die neue Form eine deutlich stärkere Stimulierung der frühen Phase der Insulinsekretion (0–40 Minuten) als die entsprechende Standardmedikation. In der Zeit danach (40–300 Minuten) besteht ein Trend zur Verringerung der Insulinspiegel. Damit ist gezeigt, daß die Stimulierung nur der früheren Insulinsekretion im Sinne des Therapieziels nicht nur bei Stoffwechselgesunden sondern auch bei der Zielgruppe – den Typ-2-Diabetikern – erreicht wird.

Tabelle 8
Teilflächen unter der Plasmainsulinkurve von 7 Typ-2-Diabetikern während eines Standard-Frühstücks, Mittelwert ± SEM (Prozent der Basalfläche).

| | 0–40 Minuten | 40–300 Minuten |
|---|---|---|
| Standard-medikation | 190 ± 14 | 424 ± 53 |
| Erfindungsgemäßes Präparat | 318 ± 43 | 378 ± 53 |

Die vorstehend genannten Befunde zeigen, daß die medizinische Zielsetzung:

a) Vermeidung eines unphysiologischen Blutzuckeranstieges nach Nahrungsaufnahme,

b) Vermeidung eines massiven Blutzuckerabfalles einige Stunden nach der Nahrungsaufnahme

c) frühzeitige, kurzanhaltende Insulinausschüttung während der Nahrungsaufnahme,

mit den erfindungsgemäßen Gliquidon-haltigen Präparaten erreicht wird.

Abbildung 8 zeigt den Verlauf der Plasmaspiegel von Gliquidon nach Gabe zweier Formen, die als basischen Hilfsstoff L-Lysin enthalten. Es zeigt sich, daß die Rezeptur analog Beispiel 23b nur relativ niedrige Plasmaspiegel zur Folge hat, während bei Gabe von Formen gemäß Beispiel 23c ausgezeichnete Ergebnisse erzielt werden. Dies beweist, daß für ausreichende in vivo Freisetzung des Wirkstoffs ein deutlicher Überschuß an basischem Hilfsstoff erforderlich ist, d. h. der positive Effekt kann nicht nur auf Salzbildung zurückgeführt werden.

Abbildung 9 zeigt, daß die anfangs erwähnten Anforderungen an ein optimales Arzneimittel für Diabetiker mit verschiedenen Rezepturen (Beispiele 23c, 24b, 25) erreicht werden kann. Es muß allerdings in jedem Einzelfall die Menge an Hilfsstoffen individuell optimiert werden.

Es wurde weiterhin untersucht, ob auch mit dem Wirkstoff Glibenclamid, der in der Handelsform Euglucon N kein ideales Profil der Blutzuckerkurve aufweist, schnellerwirkende Formen möglich sind. Hierzu wurden Handelsform und eine Rezeptur analog Beispiel 36 an 8 bzw. 6 gesunden Probanden verglichen.

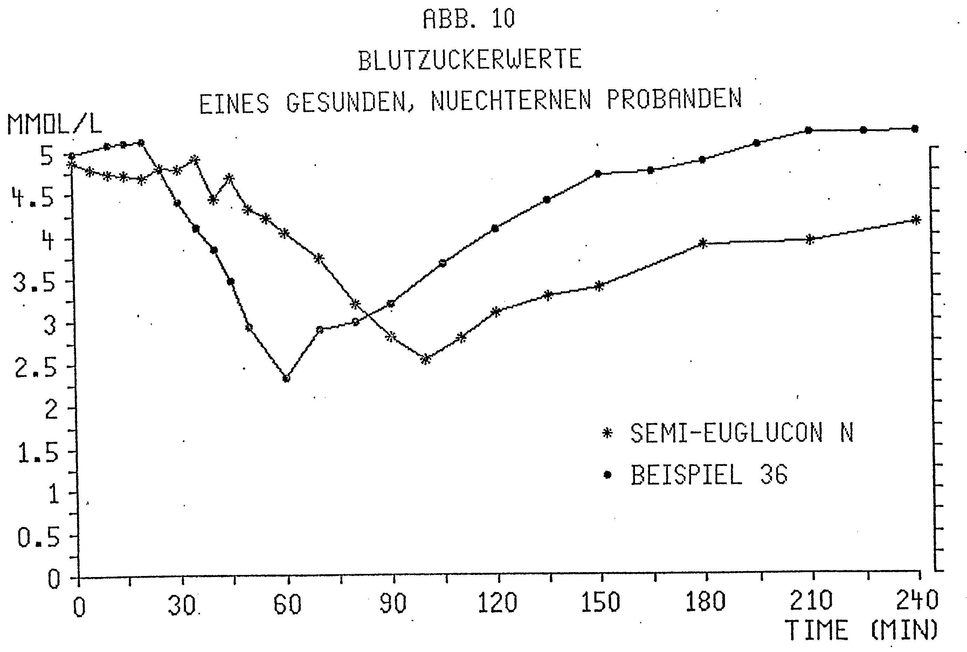

Abbildung 10 zeigt Blutzuckerverläufe einer Versuchsperson, die beide Formen an verschiedenen Tagen einnahm. Es zeigt sich, daß auch für Glibenclamid ein rascherer Wirkungseintritt und eine kürzere Wirkungsdauer realisierbar sind.

Im folgenden sollen noch die angewandten Bestimmungsmethoden dargelegt werden:

Bestimmung der Blutglukose:

Der Blutzucker wurde im venösen Vollblut bestimmt. 50 µl Blut wurden mit 500 µl 0,32 m Perchlorsäure vom Eiweiß befreit. Nach Zentrifugation erfolgte die Messung der Glukose im Überstand nach der Hexokinase-Methode am Substratautomaten.

Bestimmung von Plasmainsulin

Insulin wurde aus venösem Plasma radioimmunologisch mit Hilfe der Aktivkohlemethode bestimmt.

600 µl Vollblut wurden mit 5 µl Trasylol-EDTA-Heparin-Gemisch (5 Ampullen Trasylol/Bayer, 1,2 g EDTA + Äthylendiamintetraacetat, 150 mg Heparin, 75 ml physiologische Kochsalzlösung) versetzt, zentrifugiert und im Überstand immunoreaktives Insulin gemessen. 100 µl Plasma wurden mit 100 µl $^{125}$J.-Schweineinsulin (Novo) gelöst, in Phosphatpuffer nach Soerensen, und 250 µl Anti-Schweineinsulin-Meerschweinchenserum M 8309 (Novo)

23 Stunden bei 4 °C inkubiert. Danach wurde mit Aktivkohle (Norit/Serva) und Dextran T 70 (Pharmacia) das freie vom gebundenen Insulin abgetrennt, filtriert und im Gamma-Zähler gemessen.

Bestimmung der Plasmaspiegel:

Die Plasmaspiegel wurden mit Hilfe der HPLC bestimmt. Die Messung erfolgte in einer halbautomatischen HPLC-Anlage mit Säulenschaltung (Apparatur siehe Jour. of Chromatography, 222 (1981), 13 bis 22). Die Bestimmung erfolgte über einen externen Standard. In der analytischen Säule wird 5 µ-Reversed Phase Material (Hypersil ODS®) verwendet; für die Vorsäule Corasil C 18® mit 37 bis 50 µ. Als mobile Phase kommt ein Gemisch aus Methanol, Wasser und Piperidin (600 : 500 : 1) zur Anwendung. Die Substanz wird fluorometrisch bestimmt (Anregungswellenlänge 318 nm, Emissionswellenlänge 412 nm).

Humanuntersuchungen:

Die Blutentnahmen erfolgen über Dauerkatheter mit heparinisierten Einmalspritzen. Nach einer Vorperiode von 15 Minuten, bei der der Verlauf des Blutzuckerspiegels bzw. des Insulinspiegels ohne Medikament bestimmt wurde, wurde die galenische Zubereitung in Form eines Granulats bzw. als Tabletten in der jeweils vermerkten Dosierung mit 70 ml Wasser verabreicht.

**Patentansprüche für die Vertragsstaaten: DE, BE, FR, IT, LU, NL, SE, CH**

1. Verfahren zur Herstellung von oral antidiabetisch wirkenden Substanzen enthaltenden Zubereitungsformen, dadurch gekennzeichnet, daß sauer oder amphoter reagierende Wirkstoffe mit Hilfe basischer Hilfsstoffe in Gegenwart eines oder mehrerer lösungsvermittelnder und/oder emulgierender Stoffe, in einem Lösungsmittel gelöst werden, wobei das molare Verhältnis von Wirkstoff zu basischem Hilfsstoff kleiner als 1 : 1 sein muß, und die Lösung auf einen wasserunlöslichen Träger aufgebracht, getrocknet, und dieses Produkt gegebenenfalls noch unter Zusatz von Hilfsstoffen zu einer Arzneiform weiterverarbeitet wird.

2. Verfahren nach Anspruch 1, dadurch gekennzeichnet, daß oral antidiabetisch wirkende Sulfonylharnstoffe verwendet werden.

3. Verfahren nach Anspruch 2, dadurch gekennzeichnet, daß Gliquidon als Wirkstoff verwendet wird.

4. Verfahren nach Anspruch 1, dadurch gekennzeichnet, daß als oral antidiabetisch wirkende Substanzen

a) 4-[2-(Aroylamino)ethyl]-benzoesäuren der allgemeinen Formel

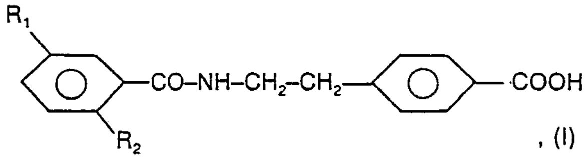

, (I)

worin $R_1$ ein Halogenatom, vorzugsweise ein Chloratom und $R_2$ eine Alkoxygruppe mit 1 bis 3 Kohlenstoffatomen, vorzugsweise die Methoxygruppe, oder eine Piperidin-1-yl- oder die Octamethyleniminogruppe bedeuten oder

b) substituierte 4-Aralkylaminocarbonylmethyl)-benzoesäuren der allgemeinen Formel

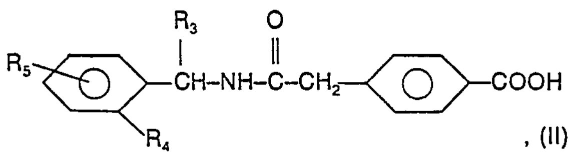

, (II)

worin $R_3$ eine Alkylgruppe mit 1 bis 4 Kohlenstoffatomen, vorzugsweise die n-Propylgruppe, oder die Phenylgruppe, $R_4$ die Piperidin-1-yl-, Pyrrolidin-1-yl oder Hexamethyleniminogruppe und $R_5$ ein Wasserstoff- oder Halogenatom oder die Methyl- oder Methoxygruppe darstellen, oder Gemische dieser Stoffe verwendet werden.

5. Verfahren nach Anspruch 1, 2, 3 und 4, dadurch gekennzeichnet, daß ein molares Verhältnis von Wirkstoff zu basischem Hilfsstoff bzw. zu einer Mischung von basischen Hilfsstoffen von 1 : 1,1 bis 1 : 10 und ein Verhältnis von Wirkstoff zu der Gesamtmenge lösungsvermittelnder Stoffe von 1 : 1 bis 1 : 10 Gewichtsteilen eingehalten wird.

6. Verfahren nach den Ansprüchen 1 bis 5, dadurch gekennzeichnet, daß als wasserunlösliche Träger hochdisperses Siliziumdioxid, mikrokristalline Cellulose, basische Aluminiumoxide, Magnesium-Aluminium-Trisilicat, quervernetztes Polyvinylpyrrolidion. Natriumcarboxymethylstärke, Tricalciumphosphat, Calciumhydrogenphosphat oder Gemische dieser Stoffe, als lösungsvermittelnde und/oder emulgierender Stoffe Polyvinylpyrrolidon, Polyethylenglykole, polyethoxyliertes Sorbitanmonooleat, Sorbit, Polyoxyethylenpolyoxypropylen-Polymere, Polyoxyethylenfettalkoholether und Glycerin-Polyethylenglykoloxystearate oder Gemische dieser Stoffe, als basische Hilfsstoffe physiologisch unbedenkliche anorganische oder organische Basen insbesondere Natronlauge, Kalilauge, Ammoniak, tert.-Natriumphosphat, Diethanolamin, Ethylendiamin, L-Lysin oder N-Methylglucamin verwendet werden.

7. Zubereitungsformen, die oral antidiabetisch wirkende Substanzen enthalten, gekennzeichnet durch einen wasserunlöslichen Träger, auf den ein Gemisch bestehend aus sauer oder amphoter reagierenden Wirkstoffen zusammen mit basisch reagierenden Hilfsstoffen im molaren Verhältnis von Wirkstoff zu basischem Hilfsstoff von kleiner als 1 : 1, und einem oder mehreren lösungsvermittelnden und/oder emulgierenden Stoffen und einem Lösungsmittel aufgebracht ist und der getrocknete Träger, gegebenenfalls mit weiteren üblichen Hilfsstoffen versehen, als Arzneiform vorliegt.

8. Zubereitungsformen gemäß Anspruch 7, dadurch gekennzeichnet, daß diese neben dem Wirkstoff oder einem Wirkstoffgemisch einen basischen Hilfsstoff oder ein basisches Hilfsstoffgemisch, einen oder mehrere wasserunlösliche Träger, und einen oder mehrere lösungsvermittelnde Stoffe und, gegebenenfalls, weitere Hilfsstoffe enthalten, wobei das molare Verhältnis von Wirkstoff zu basischem Hilfsstoff kleiner als 1 : 1 sein muß.

9. Zubereitungsformen gemäß Anspruch 7, dadurch gekennzeichnet, daß als Base physiologisch verträgliche anorganische oder organische Basen, als wasserunlösliche Träger hochdisperses Siliziumdioxid, mikrokristalline Cellulose, basische Aluminiumoxide, Magnesium-Aluminium-Trisilikate, quervernetzte Polyvinylpyrrolidone, Natriumcarboxymethylstärke, Tricalciumphosphat, Calciumhydrogenphosphat oder Gemische dieser Stoffe und als lösungsvermittelnde Stoffe Polyvinylpyrrolidone, Polyethylenglykole, polyethoxylierte Sorbitanmonooleate, Sorbit, Polyoxyethylenpolyoxypropylen-Polymere, Glycerin-Polyethylen-glykoloxystearate oder Gemische dieser Stoffe enthalten sind.

10. Zubereitungsformen gemäß Anspruch 7 bis 9, dadurch gekennzeichnet, daß diese als basische Hilfsstoffe Natronlauge, Kalilauge, tert. Natriumphosphat, Ammoniak, Diethanolamin, Ethylendiamin, L-Lysin oder N-Methylglucamin im molaren Verhältnis von Wirkstoff zu basischem Hilfsstoff von 1 : 1,1 bis 1 : 10, den Wirkstoff und lösungsvermittelnde Hilfsstoffe bzw. deren Summe im Verhältnis von 1 : 1 bis 1 : 10 Gewichtsteilen und den Wirkstoff und die Trägerstoffe bzw. deren Summe im Verhältnis von 1 : 1 bis 1 :12 Gewichtsteilen enthalten.

**Patentansprüche für den Vertragsstaat: AT**

1. Verfahren zur Herstellung von oral antidiabetisch wirkenden Substanzen enthaltenden Zubereitungsformen, dadurch gekennzeichnet, daß sauer oder amphoter reagierende Wirkstoffe mit Hilfe basischer Hilfsstoffe in Gegenwart eines oder mehrerer lösungsvermittelnder und/oder emulgierender Stoffe in einem Lösungsmittel gelöst werden, wobei das molare Verhältnis von Wirkstoff zu basischem Hilfsstoff kleiner als 1 : 1 sein muß, und die Lösung auf einen wasserunlöslichen Träger aufgebracht, getrocknet, und dieses Produkt gegebenenfalls noch unter Zusatz von Hilfsstoffen zu einer Arzneiform weiterverarbeitet wird.

2. Verfahren nach Anspruch 1, dadurch gekennzeichnet, daß oral antidiabetisch wirkende Sulfonylharnstoffe verwendet werden.

3. Verfahren nach Anspruch 2, dadurch gekennzeichnet, daß Gliquidon als Wirkstoff verwendet wird.

4. Verfahren nach Anspruch 1, dadurch gekennzeichnet, daß als oral antidiabetisch wirkende Substanzen

a) 4-[2-Aroylamino)ethyl]-benzoesäuren der allgemeinen Formel

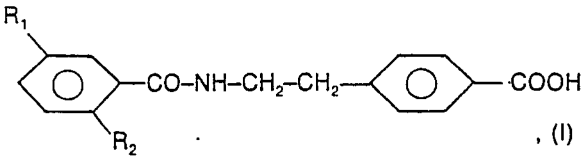

, (I)

worin $R_1$ ein Halogenatom, vorzugsweise ein Chloratom und $R_2$ eine Alkoxygruppe mit 1 bis 3 Kohlenstoffatomen, vorzugsweise die Methoxygruppe, oder eine Piperidin-1-yl- oder die Octamethyleniminogruppe bedeuten oder

b) substituierte 4-(Aralkylaminocarbonylmethyl)-benzoesäuren der allgemeinen Formel

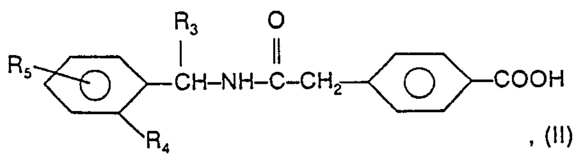

, (II)

worin $R_3$ eine Alkylgruppe mit 1 bis 4 Kohlenstoffatomen, vorzugsweise die n-Propylgruppe, oder die Phenylgruppe, $R_4$ die Piperidin-1-yl-, Pyrrolidin-1-yl- oder Hexamethyleniminogruppe und $R_5$ ein Wasserstoff- oder Halogenatom oder die Methyl- oder Methoxygruppe darstellen, oder Gemische dieser Stoffe verwendet werden.

5. Verfahren nach Anspruch 1, 2, 3 und 4, dadurch gekennzeichnet, daß ein molares Verhältnis von Wirkstoff zu basischem Hilfsstoff bzw. zu einer Mischung von basischen Hilfsstoffen von 1 : 1,1 bis 1 : 10 und ein Verhältnis von Wirkstoff zu der Gesamtmenge lösungsvermittelnder Stoffe von 1 : 1 bis 1 : 10 Gewichtsteilen eingehalten wird.

6. Verfahren nach den Ansprüchen 1 bis 5, dadurch gekennzeichnet, daß als wasserunlösliche Träger hochdisperses Siliziumdioxid, mikrokristalline Cellulose, basische Aluminiumoxide, Magnesium-Aluminium-Trisilicat, quervernetztes Polyvinylpyrrolidon, Natriumcarboxymethylstärke, Tricalciumphosphat, Calciumhydrogenphosphat oder Gemische dieser Stoffe, als lösungsvermittelnde und/oder emulgierender Stoffe Polyvinylpyrrolidon, Polyethylenglykole, polyethoxyliertes Sorbitanmonooleat, Sorbit, Polyoxyethylenpolyoxypropylen-Polymere, Polyoxyethylenfettalkoholether und Glycerin-Polyethylenglykoloxystearate oder Gemische dieser Stoffe, als basische Hilfsstoffe physiologisch unbedenkliche anorganische oder organische Basen insbesondere Natronlauge, Kalilauge, Ammoniak, tert.-Natriumphosphat, Diethanolamin, Ethylendiamin, L-Lysin oder N-Methylglucamin verwendet werden.

**Claims for the Contracting States: DE, BE, FR, IT, LU, NL, SE, CH**

1. Process for producing preparations containing substances with an oral anti-diabetic activity, characterised in that acidically or amphoterically reacting active substances are dissolved in a solvent with the aid of basic excipients in the presence of one or more solubilizing and/or emulsifying substances, whilst the molar ratio of active substance to basic excipient must be less than 1 : 1, and the solution is applied to a water-insoluble carrier and dried, and this product is further processed to produce a pharmaceutical preparation, optionally after the addition of excipients.

2. Process according to claim 1, characterised in that sulphonyl ureas with an oral anti-diabetic activity are used.

3. Process according to claim 2, characterised in that gliquidone is used as the active substance.

4. Process according to claim 1, characterised in that the substances with an oral anti-diabetic activity used are

a) 4-[2-aroylamino)ethyl]-benzoic acids of general formula

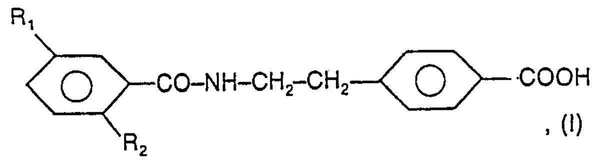

, (I)

wherein $R_1$ represents a halogen atom, preferably a chlorine atom, and $R_2$ represents a $C_{1-3}$ alkoxy, preferably a methoxy group, or a piperidin-1-yl or octamethyleneimino group or

b) substituted 4-(aralkylaminocarbonylmethyl)-benzoic acids of general formula

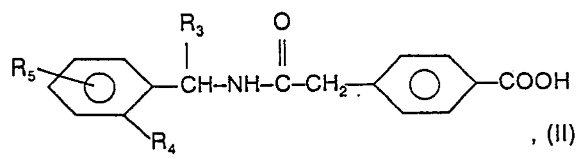

, (II)

wherein $R_3$ is a $C_{1-4}$ alkyl group, preferably an n-propyl group, or a phenyl group, $R_4$ represents a piperidin-1-yl, pyrrolidin-1-yl or hexamethylene imino group and $R_5$ represents a hydrogen or halogen or a methyl or methoxy group, or mixtures of these substances.

5. Process according to claim 1, 2, 3 and 4, characterised in that a molar ratio of active substance to basic excipient or to a mixture of basic excipients ranging from 1 : 1.1 to 1 : 10 is maintained and a ratio of active substance to the total amount of solubilizing substances ranging from 1 : 1 to 1 : 10 parts by weight is maintained.

6. Process according to claims 1 to 5, characterised in that the water-insoluble carrier used is highly dispersed silicon dioxide, microcrystalline cellulose, basic aluminium oxides, magnesium aluminium trisilicate, cross linked polyvinylpyrrolidone, sodium carboxymethyl starch, tricalcium phosphate, calcium hydrogen phosphate or mixtures of these substances, and the solubilizing and/or emulsifying substances used are polyvinylpyrrolidone, polyethylene glycols, polyethoxylated sorbitanmonooleate, sorbitol, polyoxyethylene-polyoxypropylene polymer, polyoxyethylene fatty alcohol ethers and glycerol

polyethyleneglycoloxystearates or mixtures of these substances, whilst the basic excipients used are physiologically harmless inorganic or organic bases, particularly sodium hydroxide solution, potassium hydroxide solution, ammonia, tert. sodium phosphate, diethanolamine, ethylenediamine, L-lysine or N-methylglucamine.

7. Preparations containing substances with an oral anti-diabetic activity, characterised by a water-insoluble carrier to which is applied a mixture consisting of acidically or amphoterically reacting active substances together with basically reacting excipients in a molar ratio of active substance to basic excipient of less than 1 : 1, and one or more solubilizing and/or emulsifying substances and a solvent, and the dried carrier, optionally provided with other conventional excipients, is in the form of a pharmaceutical preparation.

8. Preparations according to claim 7, characterised in that they contain, in addition to the active substance or mixture of active substances, a basic excipient or a basic mixture of excipients, one or more water-insoluble carriers and one or more solubilizing substances and optionally other excipients, whilst the molar ratio of active substance to basic excipient must be less than 1 : 1.

9. Preparations according to claim 7, characterised in that the base consists of a physiologically acceptable inorganic or organic base, the water insoluble carrier consists of a highly dispersed silicon dioxide, microcrystalline cellulose, basic aluminium oxides, magnesium-aluminium trisilicates, cross linked polyvinylpyrrolidones, sodium carboxymethyl starch, tricalcium phosphate, calcium hydrogen phosphate or mixtures of these substances and the solubilizing substances are polyvinylpyrrolidones, polyethyleneglycols, polyethoxylated sorbitanmonooleates, sorbitol, polyoxyethylenepolyoxypropylene polymer, glycerol polyethylene glycoloxystearates or mixtures of these substances.

10. Preparations according to claims 7 to 9, characterised in that they contain as basic excipients sodium hydroxide solution, potassium hydroxide solution, tert. sodium phosphate, ammonia, diethanolamine, ethylenediamine, L-lysine or N-methylglucamine in a molar ratio of active substance to basic excipient of from 1 : 1.1 to 1 : 10, they contain the active substance and solubilizing excipients or the sum thereof in a ratio of from 1 : 1 to 1 : 10 parts by weight, and they contain the active substance and the carriers or the sum thereof in a ratio of from 1 : 1 to 1 : 12 parts by weight.

**Claims for the contracting State: AT**

1. Process for producing preparations containing substances with an oral anti-diabetic activity, characterised in that acidically or amphoterically reacting active substances are dissolved in a solvent with the aid of basic excipients in the presence of one or more solubilizing and/or emulsifying substances, whilst the molar ratio of active substance to basic excipient must be less than 1 : 1, and the solution is applied to a water-insoluble carrier and dried, and this product is further processed to produce a pharmaceutical preparation, optionally after the addition of excipients.

2. Process according to claim 1, characterised in that sulphonyl ureas with an oral anti-diabetic activity are used.

3. Process according to claim 2, characterised in that gliquidone is used as the active substance.

4. Process according to claim 1, characterised in that the substances with an oral anti-diabetic activity used are

a) 4-[2-(aroylamino)ethyl]-benzoic acids of general formula

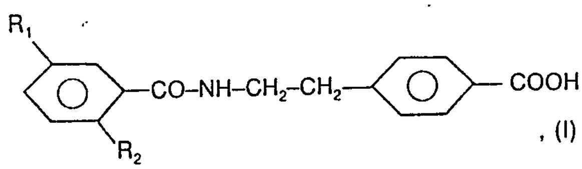

, (I)

wherein $R_1$ represents a halogen atom, preferably a chlorine atom, and $R_2$ represents a $C_{1-3}$ alkoxy, preferably a methoxy group, or a piperidin-1-yl or octamethyleneimino group or

b) substituted 4-(aralkylaminocarbonylmethyl)-benzoic acids of general formula

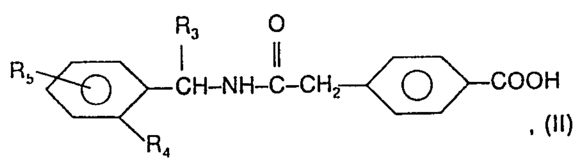

, (II)

wherein $R_3$ is a $C_{1-4}$ alkyl group, preferably an n-propyl group, or a phenyl group, $R_4$ represents a piperidin-1-yl, pyrrolidin-1-yl or hexamethylene imino group and $R_5$ represents a hydrogen or halogen atom or a methyl or methoxy group, or mixtures of these substances.

5. Process according to claim 1, 2, 3 and 4, characterised in that a molar ratio of active substance to basic excipient or to a mixture of basic excipients ranging from 1 : 1.1 to 1 : 10 is maintained and a ratio of active substance to the total amount of solubilizing substances ranging from 1 : 1 to 1 : 10 parts by weight is maintained.

6. Process according to claims 1 to 5, characterised in that the water-insoluble carrier used is highly dispersed silicon dioxide, microcrystalline cellulose, basic aluminium oxides, magnesium aluminium trisilicate, cross linked polyvinylpyrrolidone, sodium carboxymethyl starch, tricalcium phosphate, calcium hydrogen phosphate or mixtures of these substances, and the solubilizing and/or emulsifying substances used are polyvinylpyrrolidone, polyethylene glycols, polyethoxylated sorbitanmonooleate, sorbitol, polyoxyethylene-polyoxypropylene polymer, polyoxyethylene fatty alcohol ethers and glycerol polyethyleneglycoloxystearates or mixtures of these substances, whilst the basic excipients used are physiologically harmless inorganic or organic bases, particularly sodium hydroxide solution,

potassium hydroxide solution, ammonia, tert. sodium phosphate, diethanolamine, ethylenediamine, L-lysine or N-methylglucamine.

**Revendications pour les Etats contractants: DE, BE, FR, IT, LU, NL, SE, CH**

1. Procédé pour la fabrication de formes de préparations contenant des substances à activité antidiabétique par voie orale, caractérisé en ce que l'on dissout des substances actives réagissant de façon acide ou amphotère à l'aide d'adjuvants basiques en présence d'une ou de plusieurs substances de solubilisation et/ou émulsifiantes, dans un solvant, le rapport molaire de la substance active à l'adjuvant basique devant être inférieur à 1 : 1, et en ce que l'on apporte la substance sur un support insoluble dans l'eau, on sèche et on poursuit la transformation de ce produit, éventuellement en présence d'autres adjuvants, en une forme médicamenteuse.

2. Procédé selon la revendication 1, caractérisé en ce que l'on utilise des sulfonylurées à activité antidiabétique par voie orale.

3. Procédé selon la revendication 2, caractérisé en ce que l'on utilise, en tant que substance active, de la gliquidone.

4. Procédé selon la revendication 1, caractérisé en ce que l'on utilise, en tant que substances à activité antidiabétique par voie orale

a) des acides 4-[2-(aroylamino-éthyl]-benzoïques de formule générale

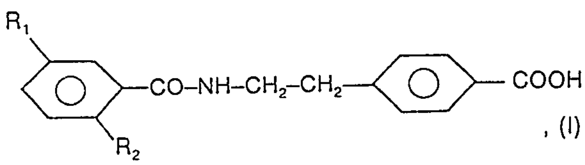

, (I)

dans laquelle $R_1$ représente un atome d'halogène, de préférence un atome de chlore et $R_2$ représente un groupe alcoxy avec 1 à 3 atomes de carbone, de préférence le groupe méthoxy, ou représente un groupe pipéridine-1-yle ou le groupe octaméthylènimino ou

b) des acides 4-(aralcoylaminocarbonylméthyl)-benzoïques de formule générale

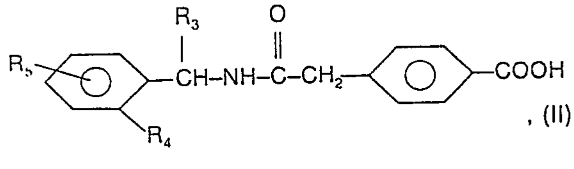

, (II)

dans laquelle $R_3$ représente un groupe alcoyle avec 1 à 4 atomes de carbone, de préférence le groupe n-propyle, ou le groupe phényle, $R_4$ représente le groupe pipéridine-1-yle, pyrrolidine-1-yle ou hexaméthylènimino et $R_5$ représente un atome d'hydrogène ou d'halogène ou le groupe méthyle ou méthoxy, ou des mélanges de ces substances.

5. Procédé selon la revendication 1, 2, 3 et 4, caractérisé en ce qu'on observe un rapport molaire de la substance active à l'adjuvant basique ou respectivement à un mélange d'adjuvants basiques de 1 : 1,1 à 1 : 10 et un rapport de la substance active à la quantité totale de substance de solubilisation de 1 : 1 à 1 : 10 parties en poids.

6. Procédé selon les revendications 1 à 5, caractérisé en ce qu'on utilise, en tant que support insoluble dans l'eau, du bioxyde de silicium hautement dispersé, de la cellulose microcristalline, de l'oxyde d'aluminium basique, du trisilicate de magnésium et d'aluminium, de la poylvinylpyrrolidone réticulée transversalement, du carboxyméthylamidon sodé, du phosphate de tricalcium, du phosphate acide de calcium ou des mélanges de ces substances, en tant que substances de solubilisation et/ou agents émulsifiants de la polyvinylpyrrolidone, des polyéthylène-glycols, du monooléate de sorbitane polyéthoxylé, du sorbitol, des polymères polyoxyéthylènepolyoxypropylène, des éthers de polyoxyéthylène et d'alcools gras, et des polyéthylène-glycoloxystéarates de glycérol ou des mélanges de ces substances, en tant qu'adjuvants basiques des bases minérales ou organiques physiologiquement inoffensives, en particulier de la lessive de soude, de la lessive de potasse, de l'ammoniaque, du phosphate trisodé, de la diéthanolamine, de l'éthylènediamine, de la L-lysine ou de N-méthylglucamine.

7. Formes de préparations qui contiennent des substances à activité antidiabétique par voie orale, caractérisées par un support insoluble dans l'eau, sur lequel est apporté un mélange constitué de substances actives réagissant de façon acide ou amphotère, ensemble avec des adjuvants réagissant de façon basique, dans le rapport molaire de la substance active à l'adjuvant basique inférieur à 1 : 1, et d'une ou plusieurs substances de solubilisation et/ou émulsifiants et d'un solvant et en ce que le support séché, éventuellement muni d'autres adjuvants usuels, se trouve sous forme médicamenteuse.

8. Formes de préparations selon la revendication 7, caractérisées en ce qu'elles contiennent, outre la substance active ou un mélange de substances actives, un adjuvant basique ou un mélange d'adjuvants basique, un ou plusieurs supports insolubles dans l'eau, et une ou plusieurs substances de solubilisation et, éventuellement, d'autres adjuvants, le rapport molaire de la substance active à l'adjuvant basique devant être inférieur à 1 : 1.

9. Formes de préparations selon la revendication 7, caractérisées en ce que sont contenues, en tant que bases, des bases minérales ou organiques physiologiquement supportables, en tant que supports insolubles du bioxyde de silicium hautement dispersé, de la cellulose microcristalline, de l'oxyde d'aluminium basique, du trisilicate de magnésium et d'aluminium, de la polyvinylpyrrolidone transversalement réticulée, du carboxyméthylamidon sodé, du phosphate de tricalcium, du phosphate acide de calcium ou des mélanges de ces substances et en tant que substances de solubilisation, des polyvinylpyrrolidones, des polyéthylène-glycols, des monooléates de sorbitane polyéthoxylés, du sorbitol, des polymères po-

lyoxyéthylènepolyoxypropylène, des glycoloxystérate de glycérol-polyéthylène, ou des mélanges de ces substances.

10. Formes de préparations selon les revendications 7 à 9, caractérisées en ce qu'elles contiennent, en tant qu'adjuvants basiques, de la lessive de soude, de la lessive de potasse, du phosphate trisodé, de l'ammoniaque, de la diéthanolamine, de l'éthylènediamine, de la L-lysine ou de la N-méthylglucamine dans le rapport molaire de la substance active à l'adjuvant basique de 1 : 1,1 à 1 : 10, la substance active et les adjuvants de solubilisation ou respectivement leur somme dans le rapport de 1 : 1 à 1 : 10 parties en poids et la substance active et les substances de support ou respectivement leur somme dans le rapport de 1 : 1 à 1 : 12 parties en poids.

**Revendications pour l'Etat contractant: AT**

1. Procédé pour la fabrication de formes de préparations contenant des substances à activité antidiabétique par voie orale, caractérisé en ce que l'on dissout des substances actives réagissant de façon acide ou amphotère à l'aide d'adjuvants basiques en présence d'une ou de plusieurs substances de solubilisation et/ou émulsifiantes, dans un solvant, le rapport molaire de la substance active à l'adjuvant basique devant être inférieur à 1 : 1, et en ce que l'on apporte la substance sur un support insoluble dans l'eau, on sèche et on poursuit la transformation de ce produit, éventuellement en présence d'autres adjuvants, en une forme médicamenteuse.

2. Procédé selon la revendication 1, caractérisé en ce que l'on utilise des sulfonylurées à activité antidiabétique par voie orale.

3. Procédé selon la revendication 2, caractérisé en ce que l'on utilise, en tant que substance active, de la gliquidone.

4. Procédé selon la revendication 1, caractérisé en ce que l'on utilise, en tant que substances à activité antidiabétique par voie orale

a) des acides 4-[2-(aroylamino)éthyl]-benzoïques de formule générale

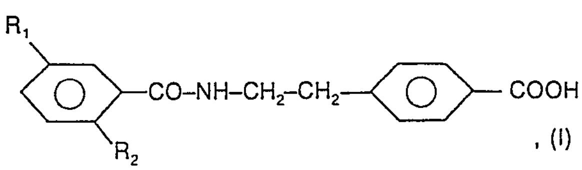

, (I)

dans laquelle $R_1$ représente un atome d'halogène, de préférence un atome de chlore et $R_2$ représente un groupe alcoxy avec 1 à 3 atomes de carbone, de préférence le groupe méthoxy, ou représente un groupe pipéridine-1-yle ou le groupe octaméthylènimino ou

b) des acides 4-(aralcoylaminocarbonylméthyl)-benzoïques de formule générale

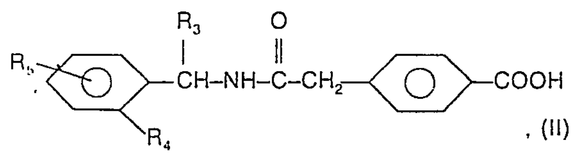

, (II)

dans laquelle $R_3$ représente un groupe alcoyle avec 1 à 4 atomes de carbone, de préférence le groupe n-propyle, ou le groupe phényle, $R_4$ représente le groupe pipéridine-1-yle, pyrrolidine-1-yle ou hexaméthylènimino et $R_5$ représente un atome d'hydrogène ou d'halogène ou le groupe méthyle ou méthoxy, ou des mélanges de ces substances.

5. Procédé selon la revendication 1, 2, 3 et 4, caractérisé en ce qu'on observe un rapport molaire de la substance active à l'adjuvant basique ou respectivement à un mélange d'adjuvants basiques de 1 : 1,1 à 1 : 10 et un rapport de la substance active à la quantité totale de substance de solubilisation de 1 : 1 à 1 : 10 parties en poids.

6. Procédé selon les revendications 1 à 5, caractérisé en ce qu'on utilise, en tant que support insoluble dans l'eau, du bioxyde de silicium hautement dispersé, de la cellulose microcristalline, de l'oxyde d'aluminium basique, du trisilicate de magnésium et d'aluminium, de la polyvinylpyrrolidone réticulée transversalement, du carboxyméthylamidon sodé, du phosphate de tricalcium, du phosphate acide de calcium ou des mélanges de ces substances, en tant que substances de solubilisation et/ou agents émulsifiants de la polyvinylpyrrolidone, des polyéthylène-glycols, du monooléate de sorbitane polyéthoxylé, du sorbitol, des polymères polyoxyéthylènepolyoxypropylène, des éthers de polyoxyéthylène et d'alcools gras, et des polyéthylène-glycoloxystéarates de glycérol ou des mélanges de ces substances, en tant qu'adjuvants basiques des bases minérales ou organiques physiologiquement inoffensives, en particulier de la lessive de soude, de la lessive de potasse, de l'ammoniaque, du phosphate trisodé, de la diéthanolamine, de l'éthylènediamine, de la L-lysine ou de N-méthylglucamine.

ABB. 1

VERGLEICH DER BLUTZUCKER-WERTE

BEI GESUNDEN, NUECHTERNEN PROBANDEN

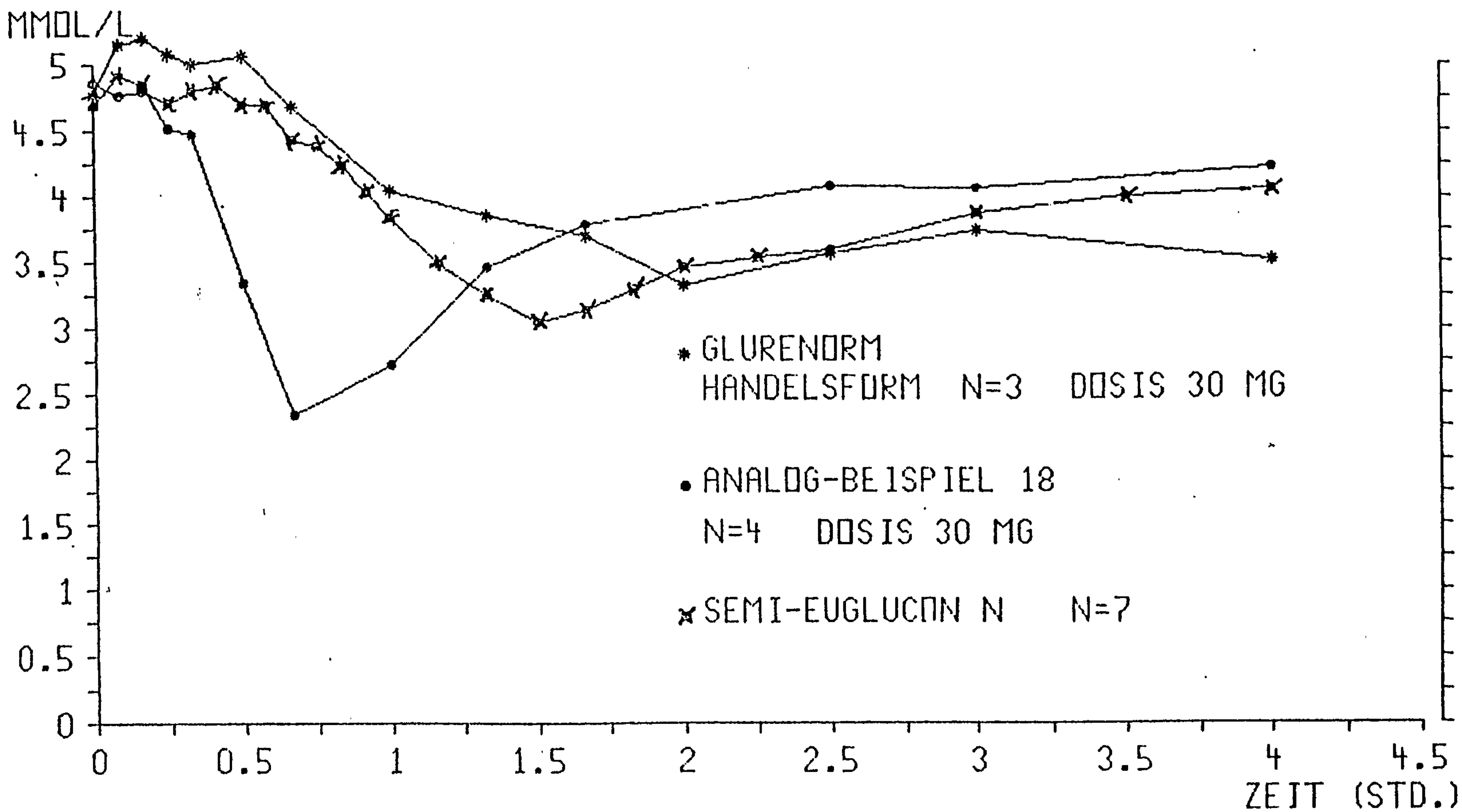

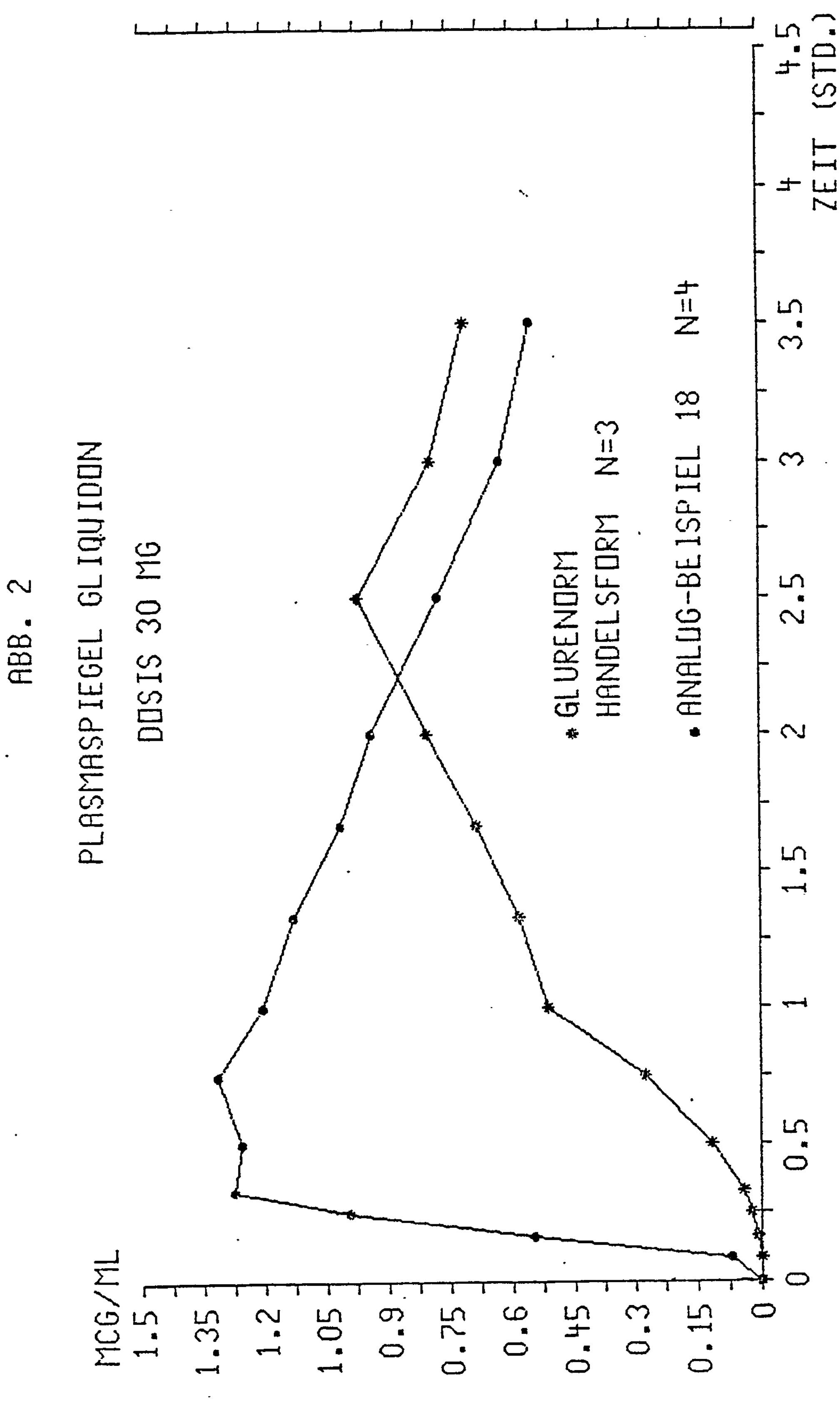
ABB. 2
PLASMASPIEGEL GLIQUIDON
DOSIS 30 MG
MCG/ML
1.5
1.35
1.2
1.05
0.9
0.75
0.6
0.45
0.3
0.15
0
0
0.5
1
1.5
2
2.5
3
3.5
4
4.5
ZEIT (STD.)
* GLURENORM
HANDELSFORM N=3
• ANALOG-BEISPIEL 18 N=4

ABB. 3

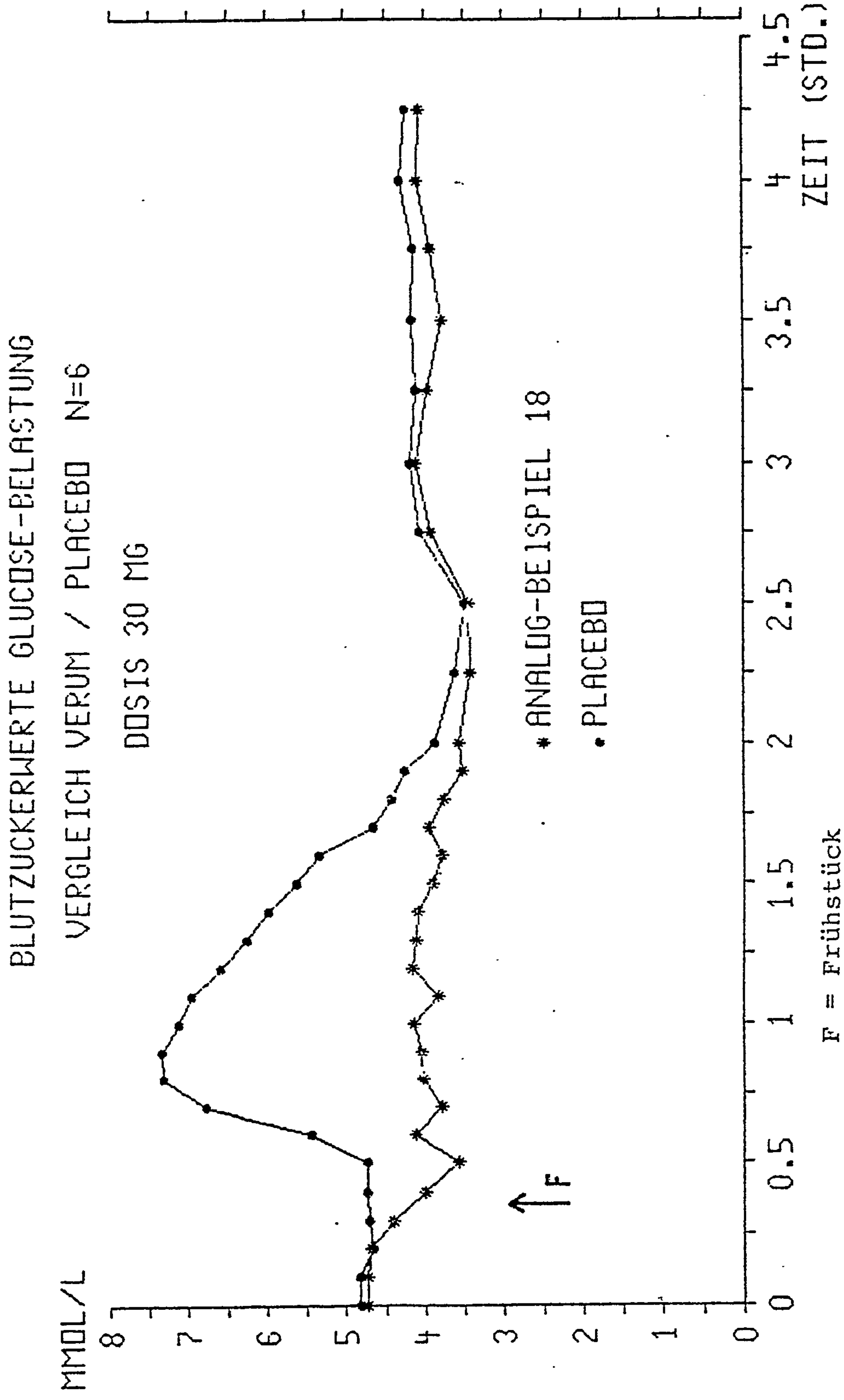
BLUTZUCKERWERTE GLUCOSE-BELASTUNG
VERGLEICH VERUM / PLACEBO N=6
DOSIS 30 MG
MMOL/L
8
7
6
5
4
3
2
1
0
0
0.5
1
1.5
2
2.5
3
3.5
4
4.5
ZEIT (STD.)
F
* ANALOG-BEISPIEL 18
• PLACEBO
F = Frühstück

ABB. 4

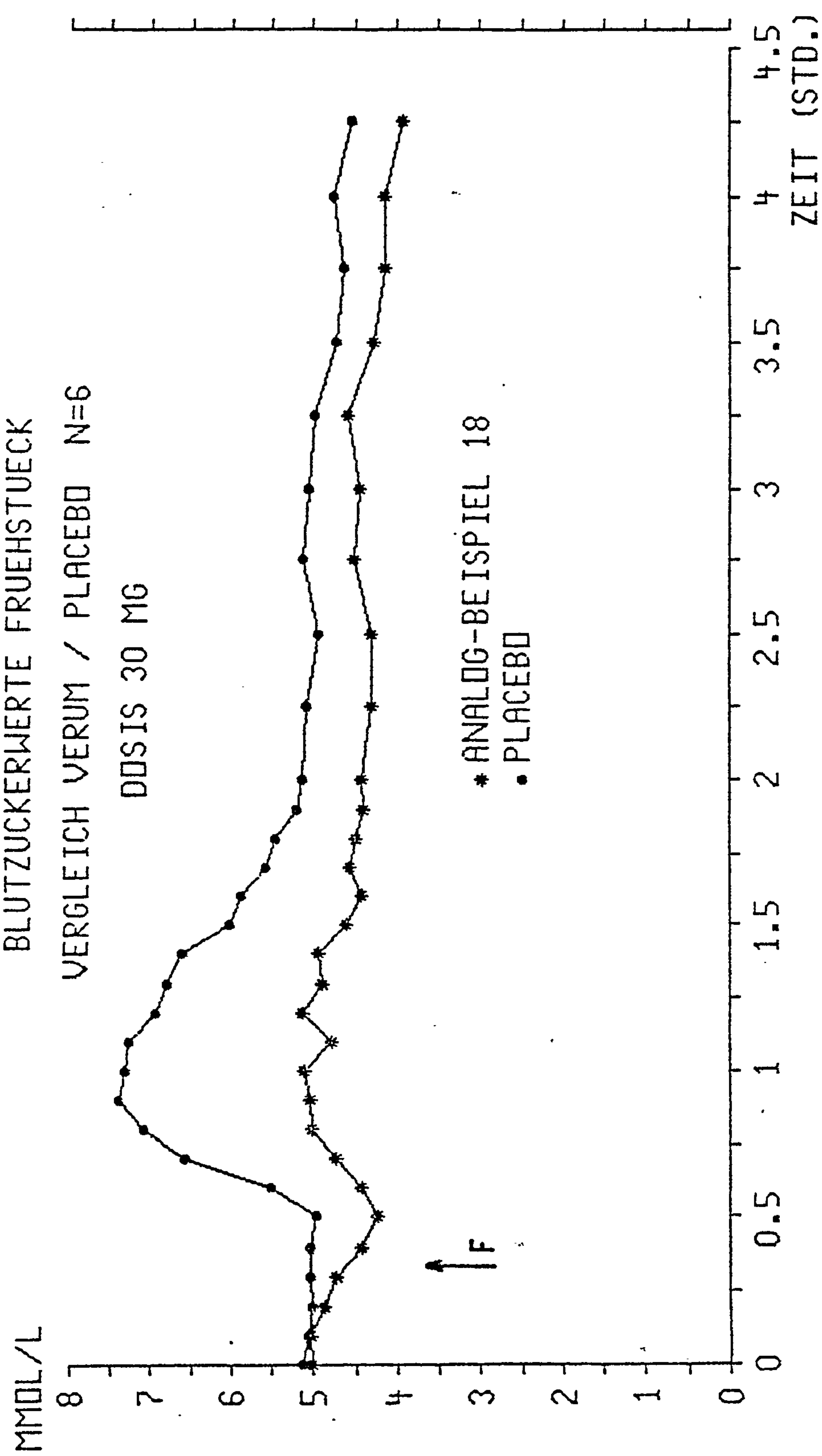
BLUTZUCKERWERTE FRUEHSTUECK
VERGLEICH VERUM / PLACEBO N=6
DOSIS 30 MG
* ANALOG-BEISPIEL 18
• PLACEBO
MMOL/L
8
7
6
5
4
3
2
1
0
F
0
0.5
1
1.5
2
2.5
3
3.5
4
4.5
ZEIT (STD.)

ABB. 5

BLUTZUCKERWERTE FRÜHSTÜCK NACH 15 MIN.

VERGLEICH VERSCHIEDENER FORMEN

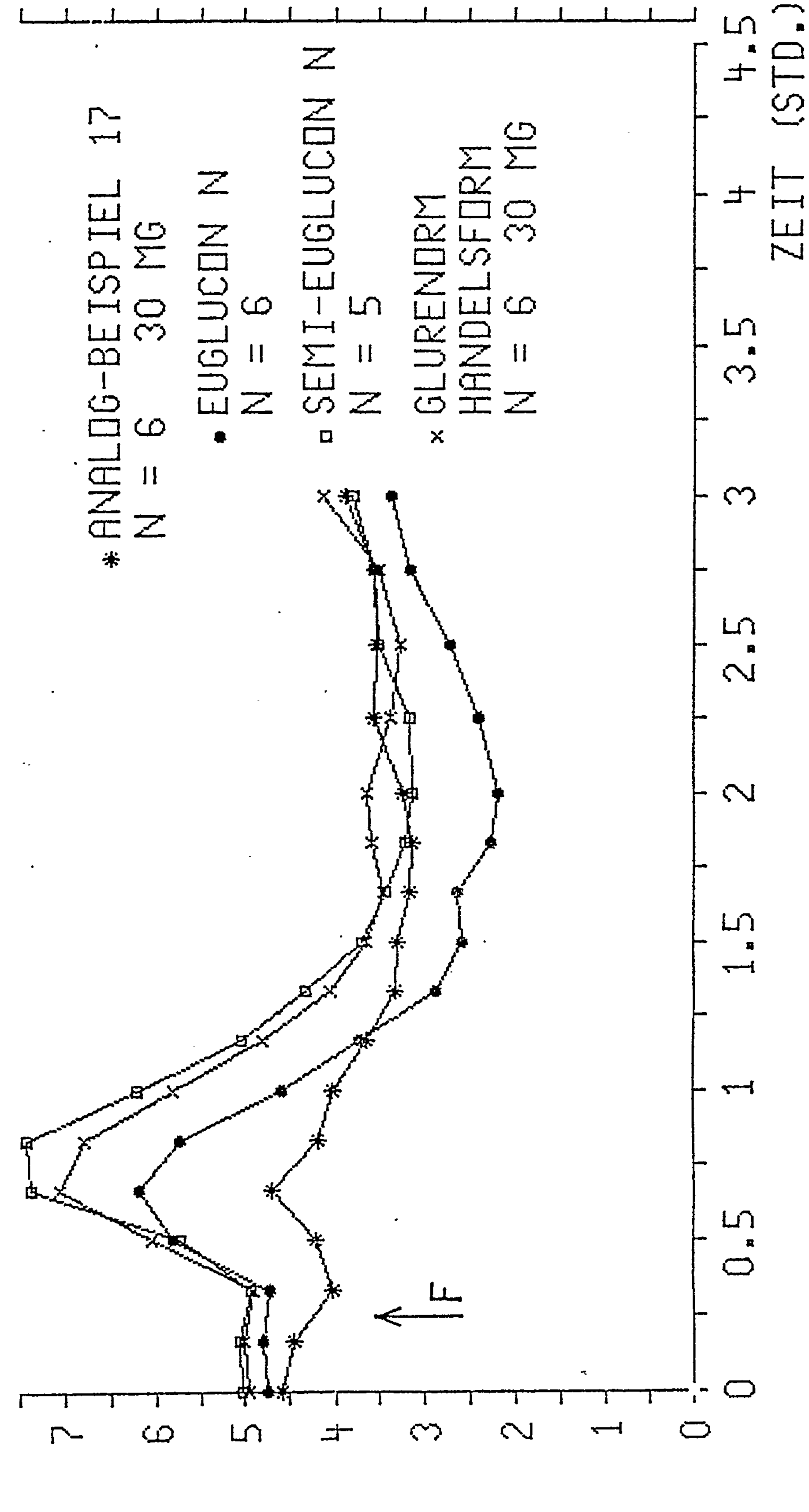

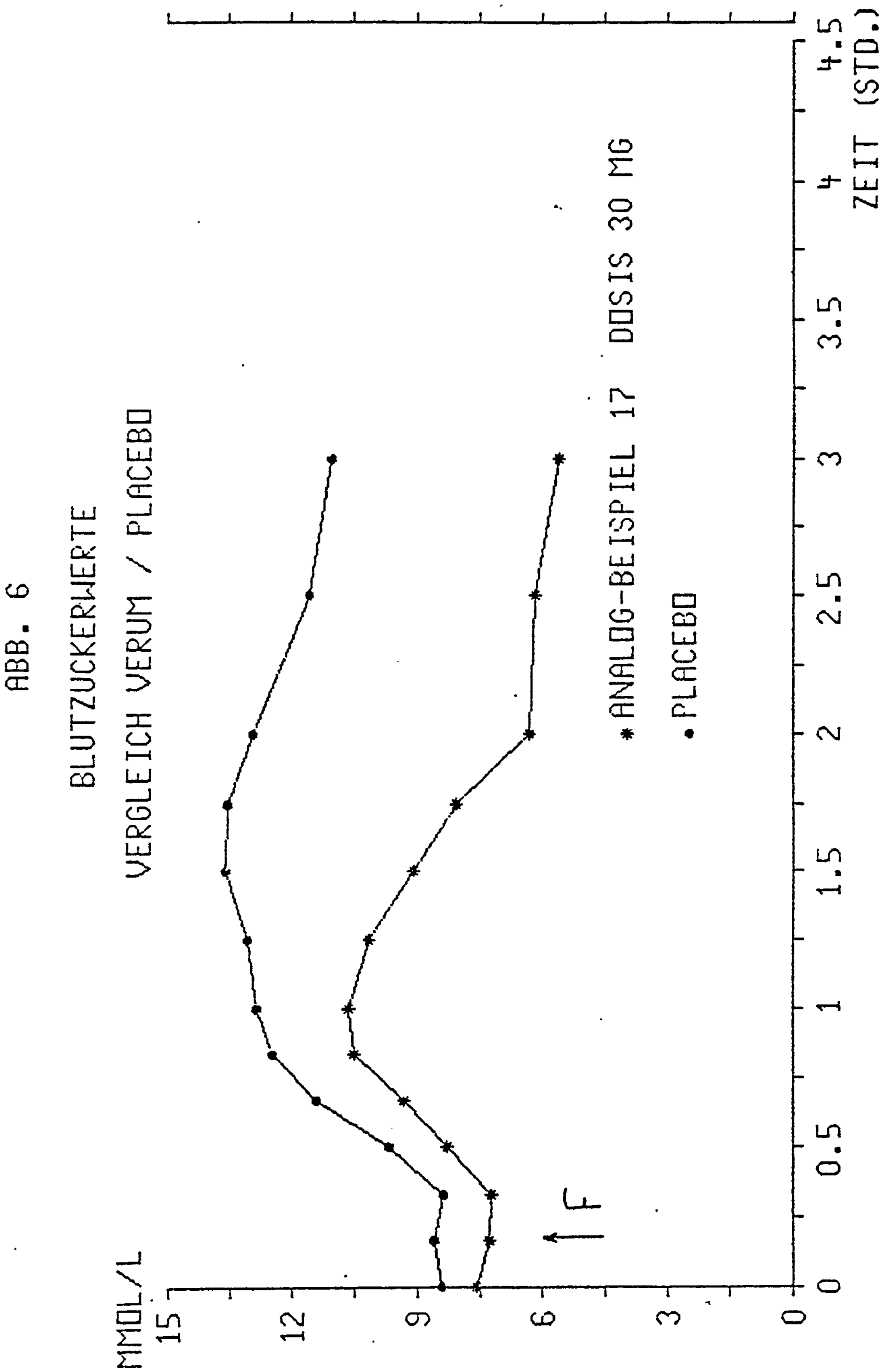
ABB. 6
BLUTZUCKERWERTE
VERGLEICH VERUM / PLACEBO
MMOL/L
15
12
9
6
3
0
F
* ANALOG-BEISPIEL 17 DOSIS 30 MG
• PLACEBO
0
0.5
1
1.5
2
2.5
3
3.5
4
4.5
ZEIT (STD.)

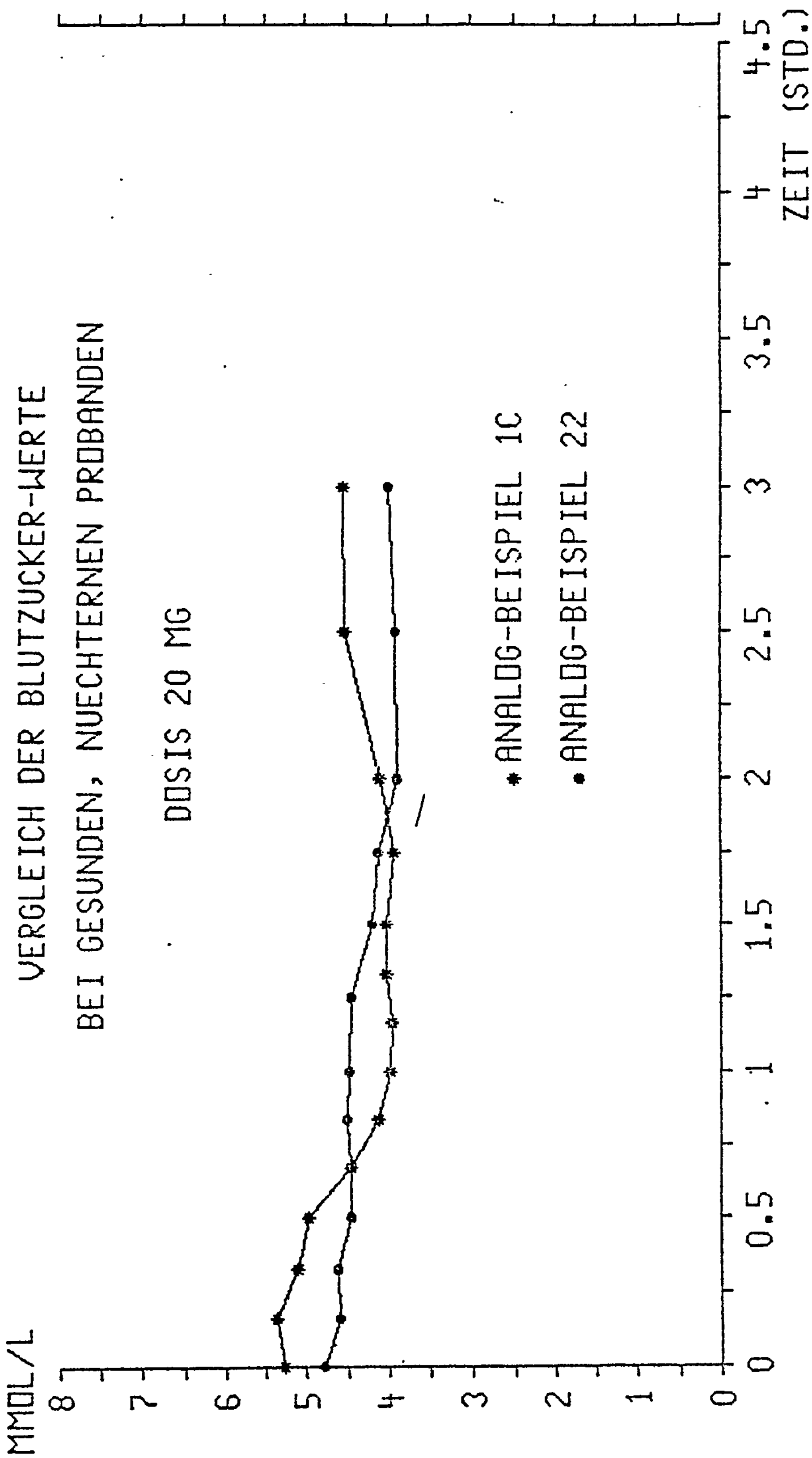
ABB. 7
VERGLEICH DER BLUTZUCKER-WERTE
BEI GESUNDEN, NUECHTERNEN PROBANDEN
DOSIS 20 MG
ANALOG-BEISPIEL 1C
ANALOG-BEISPIEL 22
MMOL/L
ZEIT (STD.)
0
0.5
1
1.5
2
2.5
3
3.5
4
4.5
8
7
6
5
4
3
2
1
0

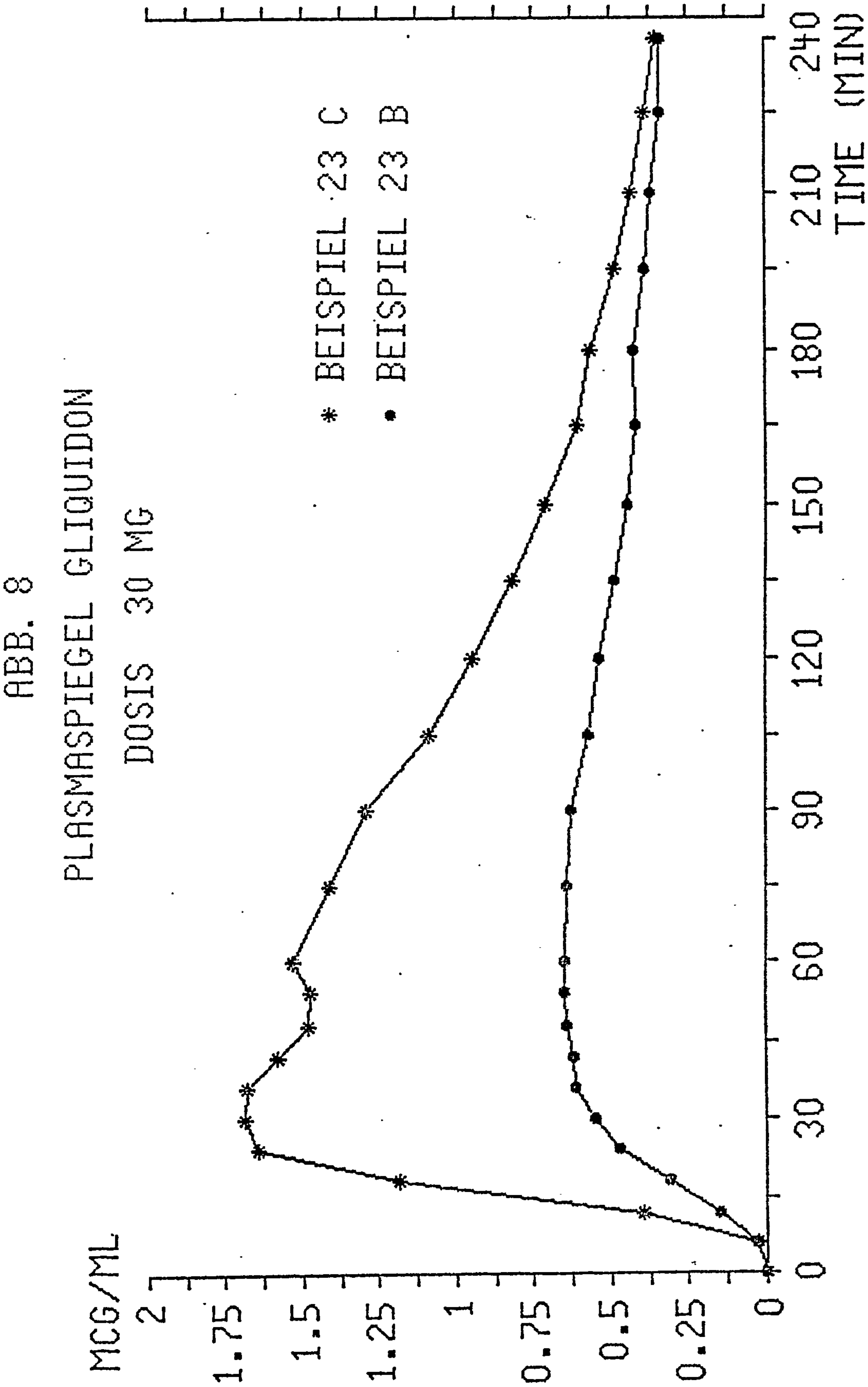


ABB. 8
PLASMASPIEGEL GLIQUIDON
DOSIS 30 MG

ABB. 9

BLUTZUCKERWERTE
BEI GESUNDEN NUECHTERNEN PROBANDEN

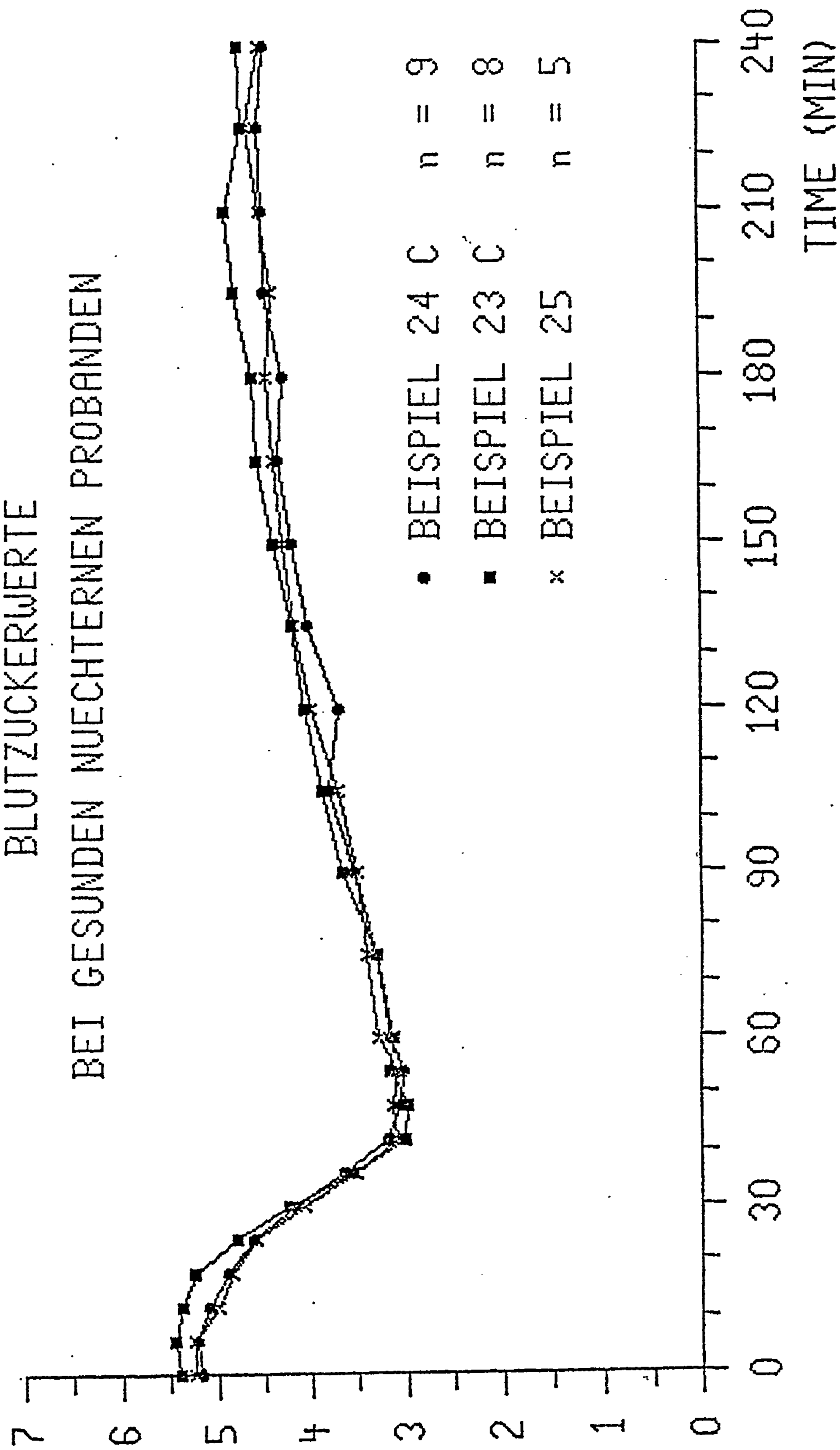

ABB. 10

BLUTZUCKERWERTE

EINES GESUNDEN, NUECHTERNEN PROBANDEN